# EUROPEAN PATENT APPLICATION

(11) **EP 2 442 394 A1**
(43) Date of publication of application: **18.04.2012**
(21) Application number: 10786233.6
(22) Date of filing: 04.06.2010
(51) Int. Cl.: H01M 8/16, C12N 1/00, C12N 1/20, G01N 27/327, H01M 4/88, H01M 4/90

(54) **FUEL CELL, PROCESS FOR MANUFACTURE OF FUEL CELL, ELECTRONIC DEVICE, ENZYME-IMMOBILIZED ELECTRODE, BIOSENSOR, ENERGY CONVERSION ELEMENT, CELL, CELL ORGANELLE, AND BACTERIUM**

(30) Priority: 08.06.2009 JP 2009137179; 19.05.2010 JP 2010115399
(71) Applicant: Sony Corporation, Tokyo 108-0075 (JP)
(72) Inventor: MATSUMOTO, Ryuhei, Tokyo 108-0075 (JP); SAKAI, Hideki, Tokyo 108-0075 (JP); TOKITA, Yuichi, Tokyo 108-0075 (JP); FUJITA, Shuji, Tokyo 108-0075 (JP)
(74) Representative: Müller - Hoffmann & Partner
(86) International application number: PCT/JP2010/059892
(87) International publication number: WO 2010/143702

(57) **Abstract**

There are provided a fuel cell and a production method therefor in which one or more types of enzymes or further coenzymes are enclosed in a micro space so that electrons can be efficiently extracted from a fuel such as glucose or the like by an enzyme reaction using the micro space as a reaction field, thereby producing electric energy, and in which the enzyme or further the coenzyme can be easily immobilized on an electrode.

Enzymes 13 and 14 and a coenzyme 15 necessary for an enzyme reaction are enclosed in liposome 12, and the liposome 12 is immobilized on a surface of an electrode composed of porous carbon or the like to form an enzyme-immobilized electrode. An antibiotic 16 is bonded to a bimolecular lipid membrane constituting the liposome 12 to form one or more pores 17 permeable to glucose. The enzyme-immobilized electrode is used as, for example, a negative electrode of a biofuel cell.

## Description

### Technical Field

The present invention relates to a fuel cell, a method for producing a fuel cell, an electronic apparatus, an enzyme-immobilized electrode, a biosensor, an energy-conversion element, cells, organelles, and bacteria. In more details, the present invention is suitable for use in a biofuel cell, a biosensor, and an energy-conversion element which use glucose as a fuel or a substrate, and various electronic apparatuses using a biofuel cell as a power supply.

### Background Art

In recent years, fuel cells (biofuel cells) using an enzyme have attracted attention (refer to, for example, Patent Literatures 1 to 12). In the biofuel cells, a fuel is separated into protons (H⁺) and electrons by decomposition with an enzyme, and fuel cells using as the fuel an alcohol such as methanol, ethanol, or the like, a monosaccharide such as glucose or the like, or a polysaccharide such as starch or the like have been developed.

For the biofuel cells, it is known that immobilization and arrangement of the enzyme on an electrode are very important. In addition, it is found that there is the need for an electron mediator functioning to transmit electrons to be effectively present together with an enzyme. There are various conventional methods for immobilizing an enzyme, but among these methods, the inventors of the present invention have mainly developed a polyion complex method in which a positively charged polymer and a negatively charged polymer are mixed with an enzyme at a proper ratio and applied to an electrode composed of porous carbon to stabilize an immobilization membrane while maintaining adhesion to the electrode, and a glutaraldehyde method.

### Citation List

### Patent Literature

PTL 1: Japanese Unexamined Patent Application Publication No. 2000-133297
PTL 2: Japanese Unexamined Patent Application Publication No. 2003-282124
PTL 3: Japanese Unexamined Patent Application Publication No. 2004-71559
PTL 4: Japanese Unexamined Patent Application Publication No. 2005-13210
PTL 5: Japanese Unexamined Patent Application Publication No. 2005-310613
PTL 6: Japanese Unexamined Patent Application Publication No. 2006-24555
PTL 7: Japanese Unexamined Patent Application Publication No. 2006-49215
PTL 8: Japanese Unexamined Patent Application Publication No. 2006-93090
PTL 9: Japanese Unexamined Patent Application Publication No. 2006-127957
PTL 10: Japanese Unexamined Patent Application Publication No. 2006-156354
PTL 11: Japanese Unexamined Patent Application Publication No. 2007-12281
PTL 12: Japanese Unexamined Patent Application Publication No. 2007-35437

### Non Patent Literature

NPL 1: "New development of liposome application - toward the development of artificial cells-" supervised by Kazunari AKIYOSHI and Kaoru TSUJII, published by NTS Inc., June 1, 2005
NPL 2: Biotechnology and Bioengineering, Vol. 81, No. 6, pp. 695-704 (2003)
NPL 3: Biophys. J., 71, pp. 2984-2995 (1996)

### Summary of Invention

### Technical Problem

However, the above-described immobilization method using a polyion complex greatly depends on the physicochemical properties of an enzyme, particularly the electric charge, and causes the concern that an immobilization state changes with changes in an external solution, changes in an operating environment, and the like, thereby easily causing elusion of the immobilized enzyme and the like. In addition, enzymes generally have low resistance against heat, but when enzymes are modified for practical application of biofuel cells, it is necessary to optimize a method for forming an immobilization membrane each time when the physicochemical properties of an enzyme are changed, thereby causing complexity. Further, when it is desired to extract more electrons from a fuel, a larger amount of enzyme is required, but a great deal of labor is consumed for optimization of immobilization conditions for immobilizing the enzyme.

Accordingly, a problem to be solved by the invention is to provide a fuel cell and a production method therefor in which one or more types of enzymes or further a coenzyme are enclosed in a micro space so that electrons can be efficiently extracted from a fuel such as glucose or the like by an enzyme reaction using the micro space as a reaction field, thereby producing electric energy, and in which the enzyme or further the coenzyme can be easily immobilized on an electrode.

Another problem to be solved by the invention is to provide a high-performance electronic apparatus using the above-described excellent fuel cell.

A further problem to be solved by the invention is to provide an enzyme-immobilized electrode, a high-efficiency biosensor, and an energy-conversion element which are suitable for application to the fuel cell.

A further problem to be solved by the invention is to provide cells, organelles, and bacteria capable of generating electric energy by efficiently extracting electrons from glucose used as a substrate.

### Solution to Problem

The inventors of the present invention performed intensive research for solving the above-mentioned problems. As a result, it was found that when an enzyme or further a coenzyme necessary for enzyme reaction are enclosed in liposome which is an artificial cell in a biofuel cell, it is possible to efficiently effect an enzyme reaction to produce a very high catalyst current and make easy immobilization on an electrode as compared with the case in which the same amounts of an enzyme or further a coenzyme are used without being enclosed in liposome. In addition, the effectiveness of this method was experimentally confirmed, and researches were made for an applicable range of this method from various viewpoints, leading to the achievement of the present invention. Also, this method is suitable for application to not only the biofuel cell but also various elements or apparatuses using enzymes or further using coenzymes.

The conventional established theory is overthrown by the finding uniquely obtained by the inventors that when an enzyme or further a coenzyme necessary for enzyme reaction are enclosed in liposome, an enzyme reaction can be far more efficiently effected to produce a very high catalyst current. Namely, it is conventionally considered that when an enzyme enclosed in liposome is regarded as a biocatalyst, the reaction rate is low because the permeation rate of a substrate to a bimolecular lipid membrane (lipid bilayer) constituting liposome is limited. For example, in Non Patent Literature 1, it is described in lines 2 to 6 in the right column on page 454 that when an enzyme enclosed in liposome is used as a biocatalyst, there is the problem of excessively limiting the reactivity of the enzyme enclosed in liposome to a hydrophilic or high-molecular-weight substrate added to an aqueous phase outside liposome because of the high permselectivity of a lipid membrane. In addition, in Non Patent Literature 2, it is described in lines 8 to 5 from below in the right column on page 695 that the reactivity of an enzyme enclosed in liposome to a substrate added externally significantly depends on the substrate permeability in the transverse direction of the bilayer of liposome.

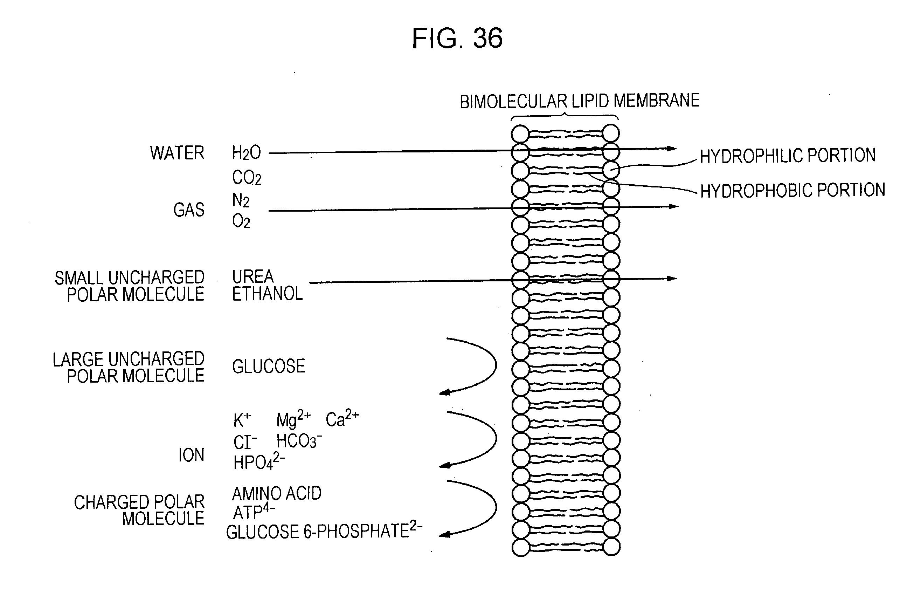

On the other hand, when glucose is used as a fuel in a biofuel cell, as already known, it is very difficult to entrap glucose in liposome from a fuel solution even by placing liposome in the fuel solution containing glucose because glucose has very low permeability to a bimolecular lipid membrane constituting liposome (refer to Fig. 36). As a result of intensive research for this point, the inventors found that the problem can be resolved by forming a pore permeable to glucose in the bimolecular lipid membrane constituting liposome, leading to the achievement of the present invention.

That is, in order to solve the above problems, the present invention provides a fuel cell configured to have a structure in which a positive electrode and a negative electrode face each other with a proton conductor provided therebetween, and to extract electrons from a fuel using an enzyme,
wherein at least one type of enzyme is enclosed in liposome; and
a bimolecular lipid membrane constituting the liposome has one or more pores permeable to glucose.

In addition, in producing a fuel cell having a structure in which a positive electrode and a negative electrode face each other with a proton conductor provided therebetween, electrons being extracted from a fuel using an enzyme, the present invention provides a method for producing a fuel cell, the method including a step of forming one or more pores permeable to glucose in a bimolecular lipid membrane constituting liposome after at least one type of enzyme is enclosed in the liposome.

In the above-described invention, typically, the fuel cell is configured so as to extract electrons from a fuel using an enzyme and a coenzyme, wherein at least one type of enzyme and at least one type of coenzyme are enclosed in liposome.

Liposome is a closed vesicle made of a bimolecular lipid membrane composed of phospholipid and the like, the inside thereof including an aqueous phase. The liposome includes not only a unilamellar liposome (SUV: Small Unilamellar Vesicle, GUV: Giant Unilamellar Vesicle) composed of a single bimolecular lipid membrane but also a multilamellar liposome (MUV) having a nest including small liposomes (SUV) entrapped in giant liposome (GUV). Liposomes, for example, having a diameter of about 100 nm to as large as 10 µm can be formed. The diameter is selected according to demand, but a specific example is 2 to 7 µm. Any phospholipid may be basically used, and either glycerolipid or sphingolipid may be used. Examples of the glycerolipid include, but are not limited to, phosphatidic acid, phosphatidyl choline (lecithin), phosphatidyl ethanolamine, phosphatidyl serine, phosphatidyl glycerol, diphosphatidyl glycerol (cardiolipin), and the like. Examples of the sphingolipid include, but are not limited to, sphingomyelin and the like. A typical example of phosphatidyl choline is dimyristoylphosphatidyl choline (DMPC). The formation of liposome and enclosure of an enzyme and a coenzyme in liposome can be performed using a conventional known method, and the method is selected according to demand.

In order to form one or more pores permeable to glucose in the bimolecular lipid membrane constituting liposome, typically, an antibiotic is bonded to the bimolecular lipid membrane. As a result, a pore which passes through the bimolecular lipid membrane is formed in a form of being edged with the antibiotic. As the antibiotic, any one of various conventional known antibiotics can be used and selected according to demand. The pore is formed to have a size such that an enzyme or further a coenzyme to be enclosed in liposome is not at least easily permeated. The pore can be formed without using an antibiotic. For example, the pore may be formed by inserting a micro tube having a sufficiently small inner diameter, such as a carbon nanotube or the like, to pass through the bimolecular lipid membrane constituting liposome.

Among the enzymes or further coenzymes necessary for enzyme reaction, at least one type of enzyme or further at least one type of coenzyme is enclosed in the liposome, but all enzymes or further coenzymes necessary for enzyme reaction may be enclosed in the liposome, or without being enclosed in the liposome, part of the enzymes or the coenzymes may be incorporated or immobilized in the bimolecular lipid membrane constituting the liposome or may be present outside the liposome. When the enzyme or the coenzyme is immobilized on the bimolecular lipid membrane constituting liposome, an anchor, for example, a polyethylene glycol chain or the like, can be used.

The liposome is preferably immobilized on a negative electrode but need not be necessarily immobilized, and when an electrolyte containing a buffer solution (buffer material) is used as a proton conductor, the liposome may be contained in the buffer solution. The liposome can be immobilized by various conventional known immobilization methods used for, for example, immobilizing cells. In addition, in this case, an intermediate layer may be formed between the negative electrode and the liposome in order to stabilize the immobilization of the liposome on the negative electrode. As the intermediate layer, biopolymers such as proteins, DNA, and the like, polymer electrolytes having both the hydrophilic and hydrophobic properties, materials which can form structures such as a micelle, an inverted micelle, a lamella, and the like, and compounds having a nanometer structure, one or more properties, and high biocompatibility can be used. Usable examples of the proteins include acid proteins represented by albumin, such as alcohol dehydrogenase, lactate dehydrogenase, ovalbumin, myokinase, and the like; and lysozyme, cytochrome c, myoglobin, trypsinogen, and the like which have isoelectric points on the alkali side. The intermediate layer composed of such a protein is physically adsorbed on a surface of the electrode, so that the liposome can be stably immobilized on the negative electrode by immobilizing the liposome on the intermediate layer.

As a method for stably immobilizing the liposome on the negative electrode, it is effective to use the following method: That is, the liposome can be stably immobilized on the negative electrode by immobilizing a first material on the negative electrode, bonding a second material to the liposome, and bonding together the first material immobilized on the negative electrode and the second material bonded to the liposome. As a preferred combination of the first material and the second material, a specifically bonded pair can be used, but the combination is not necessarily limited to this. Specific examples of the combination of the first material and the second material include the followings:

### ·Avidin and biotin

Avidin is a glycoprotein which is specifically bonded to biotin. For example, the liposome can be immobilized on the negative electrode by immobilizing avidin on the negative electrode, bonding biotin to the liposome, and bonding together the avidin immobilized on the negative electrode and the biotin bonded to the liposome. Biotin may be immobilized on the negative electrode, and avidin may be bonded to the liposome. As the avidin, various types such as streptavidin, neutroavidin, and the like can be used, and mutants thereof can also be used.

### ·Antigen and antibody

An antibody is immunoglobulin which is specifically bonded to an antigen. For example, the liposome can be immobilized on the negative electrode by forming the liposome using an antigen-modified lipid, immobilizing an antibody on the negative electrode, and bonding the antibody immobilized on the negative electrode and the antigen bonded to the liposome. An antigen may be immobilized on the negative electrode, and an antibody may be bonded to the liposome.

### ·Protein A or protein G and immunoglobulin IgG

Protein A or protein G is a protein having strong affinity for immunoglobulin IgG. For example, immunoglobulin IgG is immobilized on the negative electrode, and protein A or protein G is bonded to liposome. The liposome can be immobilized on the negative electrode by bonding immunoglobulin IgG immobilized on the negative electrode and the protein A or protein G bonded to the liposome. The protein A or protein G may be immobilized on the negative electrode, and immunoglobulin IgG may be bonded to the liposome.

### ·Sugar molecule (or sugar chain-containing compound) and lectin

Lectin is a general name for sugar-binding proteins. For example, liposome is formed by mixing with transmembrane lectin, and sugar molecules (or a sugar chain-containing compound) are immobilized on a negative electrode. The liposome can be immobilized on the negative electrode by bonding sugar chains of the sugar molecules (or a sugar chain-containing compound) immobilized on the negative electrode and lectin bonded to the liposome. Lectin may be immobilized on the negative electrode, and sugar molecules (or a sugar chain-containing compound) may be bonded to the liposome.

### ·DNA and complementary strand DNA

For example, liposome is formed using a water-soluble lipid to which DNA is bonded, and complementary strand DNA is immobilized on a negative electrode. Then, the liposome can be immobilized on the negative electrode by hybridization bonding of the complementary strand DNA immobilized on the negative electrode and the DNA bonded to the liposome.

### ·Glutathione and glutathione S-transferase (GST)

For example, liposome is formed by mixing with a GST-fused transmembrane protein, and glutathione is immobilized on a negative electrode. Then, the liposome can be immobilized on the negative electrode by bonding the glutathione immobilized on the negative electrode and GST bonded to the liposome.

### ·Heparin and heparin-binding molecule

For example, a bimolecular lipid membrane constituting liposome is modified with heparin-binding molecules, and a negative electrode is modified with heparin. Then, the liposome can be immobilized on the negative electrode by bonding the heparin of the heparin-modified negative electrode and the heparin-binding molecules bonded to the liposome.

### ·Hormone and hormone receptor

A hormone receptor is a protein molecule or molecular complex which specifically receives hormone molecules. For example, a hormone receptor is immobilized on a negative electrode, a hormone is bonded to liposome, and the liposome can be immobilized on the negative electrode by bonding the hormone receptor immobilized on the negative electrode and the hormone bonded to the liposome.

### ·Carboxylic acid and imide

For example, a carboxylic acid is bonded to a negative electrode, and an amine is bonded to liposome. Then, the liposome can be immobilized on the negative electrode by bonding the carboxylic acid bonded to the negative electrode and the amine bonded to the liposome by amide coupling using an imide such as carbodiimide, N-hydroxysuccinimide, or the like. An amine may be bonded to a negative electrode, and a carboxylic acid may be bonded to liposome.

In addition, when an electrode material of the negative electrode is carbon, as a method for bonding a carboxylic acid to the negative electrode, for example, a method of refluxing or electrochemically oxidizing a diazonium salt in a solution containing nitric acid, sulfuric acid, hydrogen peroxide, or the like can be used.

As the fuel, any one of various fuels such as glucose and the like can be used and selected according to demand. Examples of the fuel other than glucose include various organic acids involved in the citric acid cycle, sugar and organic acids involved in the pentose phosphate cycle and glycolysis system, and the like. The various organic acids involved in the citric acid cycle include lactic acid, pyruvic acid, acetyl CoA, citric acid, isocitric acid, α-ketoglutaric acid, succinyl CoA, succinic acid, fumaric acid, malic acid, oxaloacetic acid, and the like. The sugar and organic acids involved in the pentose phosphate cycle and glycolysis system include glucose 6-phosphate, 6-phosphogluconolactone, 6-phosphogluconic acid, ribulose-5-phosphate, glyceryl aldehyde 3-phosphate, fructose 6-phosphate, xylilose 5-phosphate, sedoheptulose 7-phosphate, erythrose 4-phosphate, phosphoenolpyruvic acid, 1,3-bisphosphoglyceric acid, ribose 5-phosphate, and the like. Any one of these fuels can permeate through one or more glucose-permeable pores possessed by the bimolecular lipid membrane constituting liposome.

These fuels are typically used in the form of a fuel solution in which the fuel is dissolved in a conventional known buffer solution such as a phosphate buffer, a tris-buffer solution, or the like.

The enzyme enclosed in liposome typically contains an oxidase which decomposes a fuel such as glucose or the like by promoting oxidation and further contains a coenzyme oxidase which returns a coenzyme reduced with oxidation of the fuel to an oxidized form and transfers electrons to the negative electrode through an electron mediator. Specifically, the enzyme enclosed in liposome preferably contains an oxidase which decomposes a fuel such as glucose or the like by promoting oxidation and a coenzyme oxidase which returns a coenzyme reduced with the oxidase to an oxidized form. When the coenzyme is returned to an oxidized form by the action of the coenzyme oxidase, electrons are produced, and the electrons are transferred to the negative electrode from the coenzyme oxidase through the electron mediator. For example, when glucose is used as the fuel, for example, glucose dehydrogenase (GDH) (particularly NAD-dependent glucose dehydrogenase) is used as the oxidase, and for example, NAD⁺ or NADP⁺ is used as the coenzyme, and for example, diaphorase (DI) is used as the coenzyme oxidase.

As the electron mediator, any mediator can be basically used, but a compound having a quinone skeleton is preferably used. Specifically, for example, 2,3-dimethoxy-5-methyl-1,4-benzoquinone (Q0) and compounds having a naphthoquinone skeleton, e.g., various naphthoquinone derivatives such as 1-amino-1,4-naphthoquinone (ANQ), 2-amino-3-methyl-1,4-naphthoquinone (AMNQ), 2-methyl-1,4-naphthoquinone (VK3), 2-amino-3-carboxy-1,4-naphthoquinone (ACNQ), vitamin K1, and the like, can be used. As the compound having a quinone skeleton, for example, anthraquinone and its derivatives can also be used. If required, the electron mediator may contain one or two or more types of other compounds serving as an electron mediator, other than the compound having a quinone skeleton. The electron mediator may be immobilized on the negative electrode together with the liposome in which the enzyme and the coenzyme are enclosed, may be enclosed in the liposome, may be immobilized on the liposome, or may be contained in the fuel solution.

When the enzyme is immobilized on the positive electrode, the enzyme typically includes an enzyme which reduces oxygen. As the enzyme which reduces oxygen, for example, bilirubin oxidase, laccase, ascorbate oxidase, and the like can be used. In this case, in addition to the enzyme, an electron mediator is preferably immobilized on the positive electrode. As the electron mediator, for example, potassium hexacyanoferrate, potassium octacyanotungstate, and the like can be used. The electron mediator is preferably immobilized at a sufficiently high concentration, for example, 0.64 × 10⁻⁶ mol/mm² or more on an average.

As the proton conductor, any one of various conductors can be used and selected according to demand. Specific examples include cellophane, perfluorocarbonsulfonic acid (PFS) resin films, trifluorostyrene derivative copolymer films, phosphoric acid-impregnated polybenzimidazole films, aromatic polyetherketonesulfonic acid films, PSSA-PVA (polystyrenesulfonic acid polyvinyl alcohol copolymer), PSSA-EVOH (polystyrenesulfonic acid ethylenevinyl alcohol copolymer), ion-exchange resins having fluorine-containing carbonsulfonic acid groups (Nafion (trade name, US DuPont, Inc.) and the like), and the like. When an electrolyte containing a buffer solution (buffer material) is used as the proton conductor, it is preferred that the inherent buffer ability of the enzyme can be sufficiently achieved during high-output operation, and the enzyme is allowed to sufficiently exhibit its inherent ability. For this purpose, it is effective to control the concentration of the buffer material contained in the electrolyte to 0.2 M or more and 2.5 M or less, preferably 0.2 M or more and 2 M or less, more preferably 0.4 M or more and 2 M or less, still more preferably 0.8 M or more and 1.2 M or less. Any buffer substance may be used as long as pKₐ is 6 or more and 9 or less. Specific examples include dihydrogen phosphate ion (H₂PO₄⁻), 2-amino-2-hydroxymethyl-1,3-propanediol (abbreviation, tris), 2-(N-morpholino)ethanesulfonic acid (MES), cacodylic acid, carbonic acid (H₂CO₃), hydrogen citrate ion, N-(2-acetamido) iminodiacetic acid (ADA), piperazine-N,N'-bis(2-ethanesulfonic acid) (PIPES), N-(2-acetamido)-2-aminoethanesulfonic acid (ACES), 3-(N-morpholino) propanesulfonic acid (MOPS), N-2-hydroxyethylpiperazine-N'-2-ethanesulfonic acid (HEPES), N-2-hydroxyethylpiperazine-N'-3-propanesulfonic aid (HEPPS), N-[tris(hydroxymethyl)methyl]glycine (abbreviation, tricine), glycylglycine, N,N-bis(2-hydroxyethyl)glycine (abbreviation, vicine), and the like. Examples of materials which produce dihydrogen phosphate ion (H₂PO₄⁻) include sodium dihydrogen phosphate (NaH₂PO₄), potassium dihydrogen phosphate (KH₂PO₄), and the like. As the buffer substance, a compound containing an imidazole ring is also preferred. Specific examples of the compound containing an imidazole ring include imidazole, triazole, pyridine derivatives, bipyridine derivatives, imidazole derivatives (histidine, 1-methylimidazole, 2-methylimidazole, 4-methylimidazole, 2-ethylimidazole, ethyl imidazole-2-carboxylate, imidazole-2-carboxyaldehyde, imidazole-4-carboxylic acid, imidazole-4,5-dicarboxylic acid, imidazol-1-yl-acetic acid, 2-acetylbenzimidazole, 1-acetylimidazole, N-acetylimidazole, 2-aminobenzimidazole, N-(3-aminopropyl)imidazole, 5-amino-2-(trifluoromethyl)benzimidazole, 4-azabenzimidazole, 4-aza-2-mercaptobenzimidazole, benzimidazole, 1-benzylimidazole, and 1-butylimidazole), and the like. If required, in addition to the buffer substance, for example, at least one acid selected from the group consisting of hydrochloric acid (HCl), acetic acid (CH₃COOH), phosphoric acid (H₃PO₄), and sulfuric acid (H₂SO₄) may be added as a neutralizing agent. This can maintain the activity of the enzyme at a higher level. The pH of the electrolyte containing the buffer substance is preferably close to 7 but may be generally any one of 1 to 14.

Various materials can be used as electrode materials of the positive electrode and the negative electrode, and, for example, carbon materials such as porous carbon, carbon pellets, carbon felt, carbon paper, and the like can be used. As an electrode material, a porous conductive material containing a skeleton composed of a porous material and a material which covers at least a portion of the skeleton and which contains a carbon-based material as a main component can also be used (refer to Patent Literature 12).

The fuel cell can be used for almost all applications requiring electric power, for example, an electronic apparatus, movable bodies (an automobile, a two-wheeled vehicle, an aircraft, a rocket, a spacecraft, and the like), a power unit, a construction machine, a machine tool, a power system, a cogeneration system, and the like, regardless of size, and the output, size, shape, fuel type, and the like are determined according to application.

Also, the present invention provides an electronic apparatus including:
one or more fuel cells;
at least one of the fuel cells is configured:
   to have a structure in which a positive electrode and a negative electrode face each other with a proton conductor provided therebetween, and to extract electrons from a fuel using an enzyme,
   wherein at least one type of enzyme is enclosed in liposome; and
   a bimolecular lipid membrane constituting the liposome has one or more pores permeable to glucose.

The electronic apparatus may be basically any one of the types including a portable type and a stationary type, but specific examples thereof include a cellular phone, mobile apparatuses (a portable digital assistance (PDA) and the like), a robot, a personal computer (including both a desktop type and a notebook-size type), a game apparatus, a camcorder (video tape recorder), automobile-installed equipment, house electric appliances, industrial products, and the like.

The above description about the fuel cell and the method for producing the fuel cell of the invention applies to the electronic apparatus of the invention as long as it is not contrary to the properties.

In addition, the present invention provides an enzyme-immobilized electrode,
wherein liposome in which at least one type of enzyme is enclosed is immobilized; and
a bimolecular lipid membrane constituting the liposome has one or more pores permeable to glucose.

The above description about the fuel cell and the method for producing the fuel cell of the invention applies to the enzyme-immobilized electrode of the invention as long as it is not contrary to the properties.

In addition, the present invention provides a biosensor including an enzyme,
wherein at least one type of enzyme is enclosed in liposome; and
a bimolecular lipid membrane constituting the liposome has one or more pores permeable to glucose.

The above description about the fuel cell and the method for producing the fuel cell of the invention applies to the biosensor of the invention as long as it is not contrary to the properties.

In addition, the present invention provides an energy-conversion element,
wherein at least one type of enzyme is enclosed in liposome; and
a bimolecular lipid membrane constituting the liposome has one or more pores permeable to glucose.

Here, the energy-conversion element is an element which converts chemical energy possessed by a fuel or a substrate to electric energy by an enzyme reaction, and the above-described fuel cell, i.e., the biofuel cell, is one type of the energy conversion element.

The above description about the fuel cell and the method for producing the fuel cell of the invention applies to the energy-conversion element of the invention as long as it is not contrary to the properties.

In addition, the present invention provides cells,
wherein a bimolecular lipid membrane constituting a cell membrane has one or more pores permeable to glucose.

In addition, the present invention provides organelles,
wherein a bimolecular lipid membrane constituting a cell membrane has one or more pores permeable to glucose.

In addition, the present invention provides bacteria,
wherein a bimolecular lipid membrane constituting a cell membrane has one or more pores permeable to glucose.

The cells, organelles, and bacteria are not particularly limited as long as they have a metabolic system of a fuel or a substrate such as glucose or the like, and include conventional known various cells, organelles, and bacteria. If required, like in the above-described fuel cell, an enzyme or further a coenzyme necessary for decomposition of the fuel or the substrate, such as glucose or the like, may be enclosed in the cells, organelles, and bacteria.

In the invention configured as described above, at least one type of enzyme involved in an enzyme reaction or further at least one type of coenzyme is enclosed in the liposome while maintaining high activity, and thus the enzyme reaction is efficiently effected using as a reaction field the micro space in the liposome, thereby permitting efficient extraction of electrons from the fuel or the substrate such as glucose or the like. In this case, the concentration of the enzyme and the coenzyme enclosed in the liposome is very high, and thus the distance between the enzyme and the coenzyme is very small, thereby allowing a catalyst cycle due to the enzyme and the coenzyme to proceed at a very high rate and allowing the enzyme reaction to proceed at a high rate. In addition, by immobilizing the liposome on the negative electrode or the electrode, the enzyme or further the coenzyme can be easily immobilized on the negative electrode or the electrode through the liposome. Therefore, the electrons extracted from the fuel or the substrate, such as glucose or the like, can be securely transferred to the negative electrode or the electrode. In this case, the liposome can be simply immobilized as compared with immobilization of the enzyme and coenzyme with a polyion complex or the like.

In the cells, organelles, and bacteria, the enzyme reaction is efficiently effected using as reaction fields the micro-spaces in the cells, organelles, and bacteria, and thus electrons can be efficiently extracted from the fuel or the substrate, such as glucose or the like. In this case, the enzyme reaction is effected by metabolic systems provided in the cells, organelles, and bacteria, but when an enzyme necessary for decomposition of a fuel or a substrate or further a coenzyme are enclosed in the cells, organelles, and bacteria, the enzyme reaction can be effected with the enzyme and the coenzyme.

### Advantageous Effects of Invention

According to the present invention, it is possible to realize a high-efficiency fuel cell in which an enzyme reaction can be effected using as a reaction field a micro-space in liposome, in which an enzyme or further a coenzyme are enclosed, and thus electric energy can be generated by efficiently extracting electrons from a fuel or a substrate, such as glucose or the like, and in which the enzyme or further the coenzyme can easily be immobilized on an electrode. Also, a high-performance electronic apparatus can be realized using such a high-efficiency fuel cell. In addition, similarly, a high-efficiency biosensor and energy-conversion element can be realized.

In addition, cells, organelles, and bacteria capable of generating electric energy by efficiently extracting electrons from a fuel or a substrate, such as glucose or the like, can be realized.

### Brief Description of Drawings

Fig. 1 is a schematic diagram showing an enzyme-immobilized electrode according to a first embodiment of the present invention.
Fig. 2 is a schematic diagram showing liposome in which an enzyme and a coenzyme used in the enzyme-immobilized electrode according to the first embodiment of the preset invention are enclosed.
Fig. 3 is a schematic diagram schematically showing an electron transfer reaction by an enzyme and a coenzyme enclosed in liposome in the enzyme-immobilized electrode according to the first embodiment of the preset invention.
Fig. 4 is a schematic diagram for explaining the advantage of an enzyme reaction effected by enclosing an enzyme group and a coenzyme in liposome.
Fig. 5 is a photograph alternative to a drawing, showing a fluorescence microscope image of liposome in which fluorescently labeled alcohol dehydrogenase, fluorescently labeled diaphorase, and NADH are enclosed.
Fig. 6 is a photograph alternative to a drawing, showing a fluorescence microscope image of liposome in which fluorescently labeled alcohol dehydrogenase, fluorescently labeled diaphorase, and NADH are enclosed.
Fig. 7 is a photograph alternative to a drawing, showing a fluorescence microscope image of liposome in which fluorescently labeled alcohol dehydrogenase, fluorescently labeled diaphorase, and NADH are enclosed.
Fig. 8 is a schematic diagram showing the results of fluorescence monitoring of liposome in which fluorescently labeled alcohol dehydrogenase, fluorescently labeled diaphorase, and NADH are enclosed.
Fig. 9 is a schematic diagram showing the results of fluorescence monitoring of liposome in which fluorescently labeled alcohol dehydrogenase, fluorescently labeled diaphorase, and NADH are enclosed.
Fig. 10 is a schematic diagram showing the results of fluorescence monitoring of liposome in which fluorescently labeled alcohol dehydrogenase, fluorescently labeled diaphorase, and NADH are enclosed.
Fig. 11 is a schematic diagram for explaining stability of liposome.
Fig. 12 is a schematic diagram showing the results of chronoamperometry performed for a dispersion of liposome in a predetermined solution, alcohol dehydrogenase, diaphorase, and NADH being enclosed in the liposome, and the results of chronoamperometry performed for a simple dispersion of alcohol dehydrogenase, diaphorase, and NADH in a predetermined solution.
Fig. 13 is a schematic diagram showing a state of dispersion of liposome, in which alcohol dehydrogenase, diaphorase, and NADH were enclosed, in a buffer solution.
Fig. 14 is a schematic diagram showing a state of simple dispersion of alcohol dehydrogenase, diaphorase, and NADH in a buffer solution.
Fig. 15 is a schematic diagram showing the results of chronoamperometry performed for a dispersion of liposome, in which glucose dehydrogenase, diaphorase, and NADH were enclosed, in a measurement solution in Example 1 of the present invention.
Fig. 16 is a schematic diagram showing the results of chronoamperometry performed for a dispersion of liposome, in which glucose dehydrogenase, diaphorase, and NADH were enclosed, in a measurement solution in Example 1 of the present invention.
Fig. 17 is a schematic diagram showing a structural formula of amphotericin B used as an antibiotic in Example 1 of the present invention.
Fig. 18 is a sectional view showing a state in which amphotericin B is bonded to a bimolecular lipid membrane constituting liposome to form a pore in Example 1 of the present invention.
Fig. 19 is a plan view showing a state in which amphotericin B is bonded to a bimolecular lipid membrane constituting liposome to form a pore in Example 1 of the present invention.
Fig. 20 is a schematic diagram showing a biofuel cell according to a second embodiment of the present invention.
Fig. 21 is a schematic diagram schematically showing details of a configuration of a negative electrode of a biofuel cell, examples of an enzyme group and a coenzyme enclosed in liposome immobilized on the negative electrode, and an electron transfer reaction of the enzyme group and the coenzyme.
Fig. 22 is a schematic diagram showing a specific example of the configuration of the biofuel cell according to the second embodiment of the present invention.
Fig. 23 is a schematic diagram and a sectional view for explaining a structure of a porous conductive material used as an electrode material of a negative electrode in a biofuel cell according to a third embodiment of the present invention.
Fig. 24 is a schematic diagram for explaining a method for producing a porous conductive material used as an electrode material of the negative electrode in the biofuel cell according to the third embodiment of the present invention.
Fig. 25 is a schematic diagram showing a principal portion of a cell according to a fourth embodiment of the present invention.
Fig. 26 is a schematic diagram showing a mitochondrion according to a fifth embodiment of the present invention.
Fig. 27 is a schematic diagram showing a bacterium according to a sixth embodiment of the present invention.
Fig. 28 is a perspective view and a sectional view showing a porous electrode used as an electrode of a negative electrode in a biofuel cell according to a seventh embodiment of the present invention.
Fig. 29 is a schematic diagram showing an enzyme-immobilized electrode according to an eighth embodiment of the present invention.
Fig. 30 is a schematic diagram showing a sample formed for chronoamperometry measurement in Example 2 of the present invention.
Fig. 31 is a schematic diagram showing a first sample formed for comparison to Example 2 of the present invention.
Fig. 32 is a schematic diagram showing a second sample formed for comparison with Example 2 of the present invention.
Fig. 33 is a schematic diagram showing a third sample formed for comparison with Example 2 of the present invention.
Fig. 34 is a schematic diagram showing the results of chronoamperometry in Example 2 of the present invention together with the results of comparative examples.
Fig. 35 is a schematic diagram showing an enzyme-immobilized electrode according to a ninth embodiment of the present invention.
Fig. 36 is a schematic diagram showing permeabilities of various molecules to a bimolecular lipid membrane.

### Description of Embodiments

Modes for carrying out the invention (hereinafter referred to as "embodiments") are described below. In addition, description is made in the following order.
1. First embodiment (enzyme-immobilized electrode and production method therefor)
2. Second embodiment (biofuel cell)
3. Third embodiment (biofuel cell)
4. Fourth embodiment (cells)
5. Fifth embodiment (mitochondria)
6. Sixth embodiment (bacteria)
7. Seventh embodiment (biofuel cell)
8. Eighth embodiment (enzyme-immobilized electrode and production method therefor)
9. Ninth embodiment (enzyme-immobilized electrode and production method therefor)

### (1. First embodiment)

### [Enzyme-immobilized electrode]

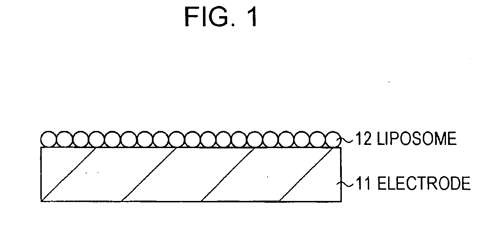

Fig. 1 shows an enzyme-immobilized electrode according to a first embodiment of the preset invention. In the enzyme-immobilized electrode, glucose is used as a substrate.

As shown in Fig. 1, in the enzyme-immobilized electrode, liposome 12 composed of a bimolecular lipid membrane of phospholipid or the like is immobilized, by physical adsorption, on a surface of an electrode 11 composed of porous carbon or the like. At least one type of enzyme involved in an intended enzyme reaction and at least one type of coenzyme are enclosed in an aqueous phase in the liposome 12.

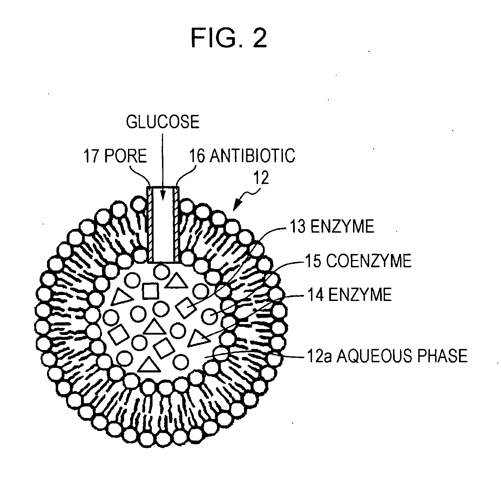

Fig. 2 shows details of a structure of the liposome 12. In Fig. 2, two types of enzymes 13 and 14 and one type of coenzyme 15 are enclosed in the aqueous phase 12a in the liposome 12. Besides, theses enzymes 13 and 14 and coenzyme 15, for example, an electron mediator may be enclosed in the aqueous phase 12a in the liposome 12. The electron mediator may be immobilized on the electrode 11 together with the liposome 12. The enzyme 13 is an oxidase which promotes oxidation of glucose used as a substrate to decompose the glucose, and the enzyme 14 is a coenzyme oxidase which returns the coenzyme 15 reduced with oxidation of the glucose to an oxidized form and transfers electrons to the electrode 11 through the electron mediator.

As shown in Fig. 2, one or a plurality of antibiotics 16 are bonded to the bimolecular lipid membrane constituting the liposome 12, forming a pore 17 passing through the bimolecular lipid membrane in a form of being edged with the antibiotic 16. Although Fig. 2 shows only one pore 17, the number and arrangement of the pores 17 are arbitrary. The size of pore 17 is selected so that permeation of glucose is possible, but permeation of the enzymes 13 and 14 and the coenzyme 15 is impossible or difficult.

As the antibiotic 16, for example, Amphotericin B, which is a polyene antibiotic, ionophore, and the like can be used, but the antibiotic 16 is not limited to these. Examples of the ionophore include valinomycin, ionomycin, nigericin, gramicidin A, monensin, carbonylcyanide-m-chlorophenylhydrazone, carbonylcyanide-p-fluoromethoxyphenylhydrazone, and the like.

### [Method for producing enzyme-immobilized electrode]

The enzyme-immobilized electrode can be produced, for example, as follows. First, the liposome 12 in which the enzymes 13 and 14 and the coenzyme 15 are enclosed is formed. Next, the antibiotic 16 is bonded to the bimolecular lipid membrane constituting the liposome 12, forming the pore 17. Next, the liposome 12 having the thus-formed pore 17 is immobilized on the electrode 11. As a result, the enzyme-immobilized electrode is produced. The pore 17 may be formed by bonding the antibiotic 16 to the bimolecular lipid membrane constituting the liposome 12 before the enzymes 13 and 14 and the coenzyme 15 are enclosed. Alternatively, the pore 17 may be formed by bonding the antibiotic 16 to the bimolecular lipid membrane constituting the liposome 12 after the liposome 12 is immobilized on the electrode 11.

More specifically, the enzyme-immobilized electrode is produced, for example, as follows. First, each of a buffer solution containing the enzyme 13, a buffer solution containing the enzyme 14, a buffer solution containing the coenzyme 15, and a buffer solution containing the liposome 12 (in which the enzymes 13 and 14 and the coenzyme 15 are not enclosed) is prepared. Next, these buffer solutions are mixed, and the mixed solution is passed through a gel filtration column to remove the enzymes 13 and 14 and the coenzyme 15 outside the liposome 12. Next, the liposome 12 in which the enzymes 13 and 14 and the coenzyme 15 are enclosed is placed in a buffer solution, and the antibiotic 16 is added to the buffer solution to bond the antibiotic 16 to the bimolecular lipid membrane constituting the liposome 12, forming the pore 17.

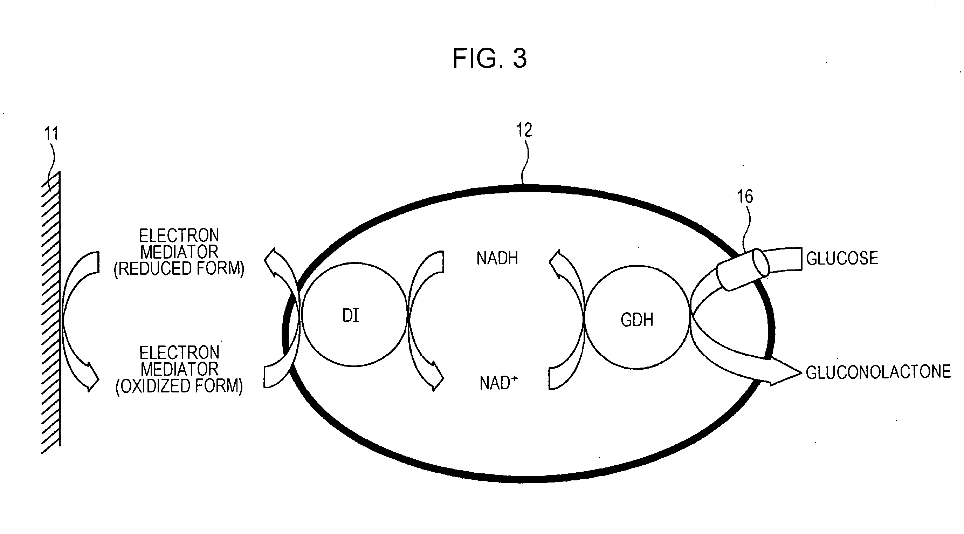

Fig. 3 schematically shows an example of an electron transfer reaction of the enzymes, the coenzyme, and the electron mediator in the enzyme-immobilized electrode. In this example, the enzyme involved in decomposition of glucose is glucose dehydrogenase (GDH). In addition, the coenzyme which produces a reduced form with oxidation reaction in the decomposition process of glucose is NAD⁺. In addition, a coenzyme oxidase which oxidizes NADH, which is the reduced form of the coenzyme, is diaphorase (DI). Further, the electron mediator receives electrons produced with oxidation of the coenzyme from the coenzyme oxidase and transfers the electrons to the electrode 11. In this case, the glucose is permeated through the pore 17 formed in the bimolecular lipid membrane constituting the liposome 12 and enters the liposome 12, producing gluconolactone by decomposition of the glucose with glucose dehydrogenase, and the gluconolactone is discharged to the outside of the liposome 12. The electron mediator enters and exits from the bimolecular lipid membrane constituting the liposome 12 to transfer electrons.

Before examples are described, description is made of the results of detailed examination of the effectiveness of use of liposome in which an enzyme and a coenzyme are enclosed as a reaction field of enzyme reaction. However, here, consideration is given to a case in which liposome with a bimolecular lipid membrane in which a pore permeable to glucose is not formed is used, and ethyl alcohol is used as a substrate of the enzyme reaction.

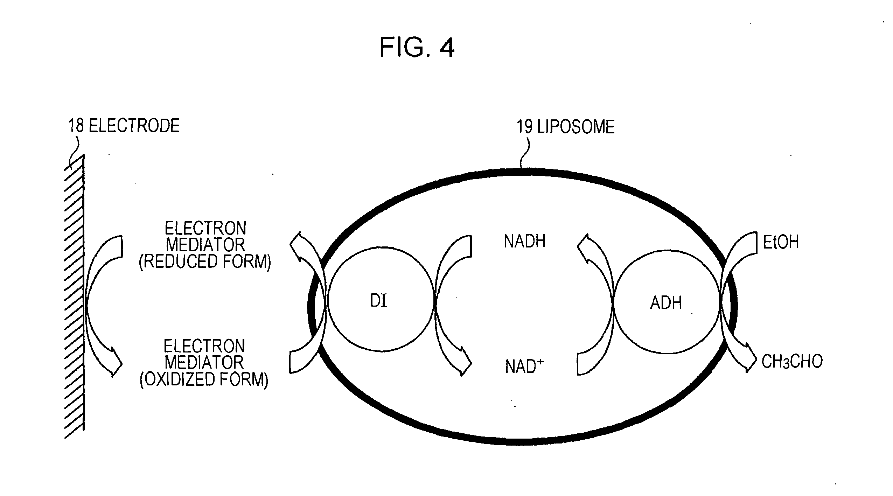

As shown in Fig. 4, an enzyme-immobilized electrode was formed by immobilizing, on an electrode 18, liposome 19 having a bimolecular lipid membrane in which a pore permeable to glucose was not formed, an enzyme and a coenzyme involved in decomposition of ethyl alcohol (EtOH) being enclosed in the liposome 19. As the electrode 18, the same as the electrode 11 was used. Fig. 4 schematically shows an example of electron transfer reaction of the enzyme, the coenzyme, and the electron mediator in the enzyme-immobilized electrode. In this example, the enzyme involved in decomposition of ethyl alcohol (EtOH) is alcohol dehydrogenase (ADH). In addition, the coenzyme which produces a reduced form with oxidation reaction in the decomposition process of ethyl alcohol is NAD⁺. In addition, a coenzyme oxidase which oxidizes NADH, which is the reduced form of the coenzyme, is diaphorase (DI). Further, the electron mediator receives electrons produced with oxidation of the coenzyme from the coenzyme oxidase and transfers the electrons to the electrode 18. In this case, ethyl alcohol is permeated through the bimolecular lipid membrane constituting the liposome 19 and enters the liposome 19, and acetaldehyde (CH₃CHO) is produced by decomposition of ethyl alcohol with the alcohol dehydrogenase, the acetaldehyde being released to the outside of the liposome 19. The electron mediator enters and exits from the bimolecular lipid membrane constituting the liposome 19 to transfer electrons.

The enzyme-immobilized electrode shown in Fig. 4 was actually formed, and an experiment was performed.

First, the enzyme-immobilized electrode was formed as follows.

Five mg of diaphorase (DI) (EC 1.8.1.4, manufactured by Amano Enzyme Inc.) was weighed and dissolved in 1 mL of a buffer solution (10 mM phosphate buffer solution, pH 7) to prepare a DI enzyme buffer solution (1).

Five mg of alcohol dehydrogenase (ADH) (NAD-dependent, EC 1.1.1.1, manufactured by Amano Enzyme Inc.) was weighed and dissolved in 1 mL of a buffer solution (10 mM phosphate buffer solution, pH 7) to prepare an ADH enzyme buffer solution (2).

The buffer solution in which each of the enzymes was dissolved is preferably refrigerated until just before dissolving, and the enzyme buffer solutions are also preferably refrigerated as far as possible.

Thirty five mg of NADH (manufactured by Sigma-Aldrich Ltd., N-8129) was weighed and dissolved in 1 mL of a buffer solution (10 mM phosphate buffer solution, pH 7) to prepare a NADH enzyme buffer solution (3).

Fifteen to three hundred mg of 2,3-dimethoxy-5-methyl-1,4-benzoquinone (Q0) was weighed and dissolved in 1 mL of a buffer solution (10 mM phosphate buffer solution, pH 7) to prepare a Q0 buffer solution (4).

One hundred mg of yolk lecithin (manufactured by Wako) was weighed and dissolved in 10 mL of a buffer solution (10 mM phosphate buffer solution, pH 7) and homogenized with a homogenizer to prepare a liposome buffer solution (5).

The amount described below was sampled from each of the solutions prepared as described above, and the samples were mixed, followed by repetition of three times of freeze thawing.
DI enzyme buffer solution (1): 50 µL
ADH enzyme buffer solution (2): 50 µL
NADH buffer solution (3): 50 µL
Liposome buffer solution (5): 50 µL

The above-described mixed solution was passed through a gel filtration column to remove the enzyme and NADH outside the liposome. The resultant liposome solution was regarded as an ADH/DI/NADH-enclosed liposome solution (6).

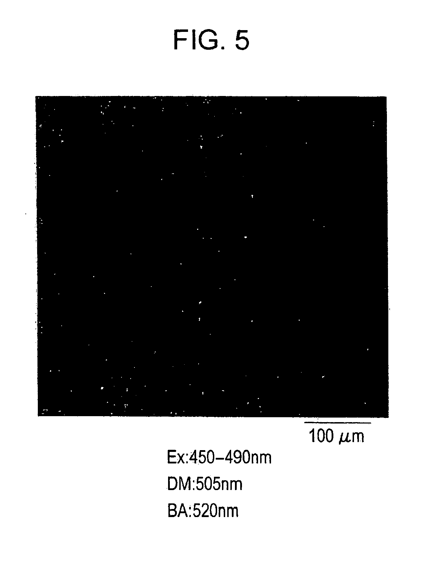

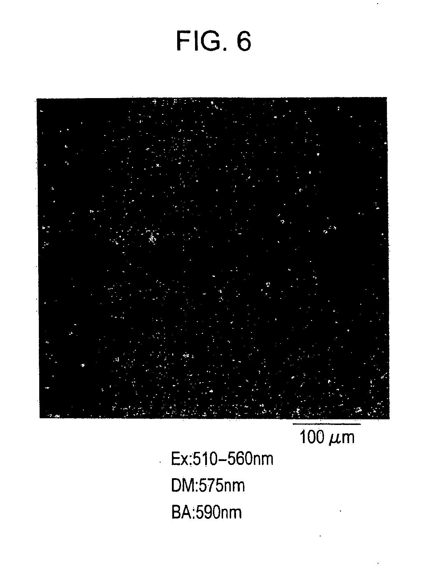

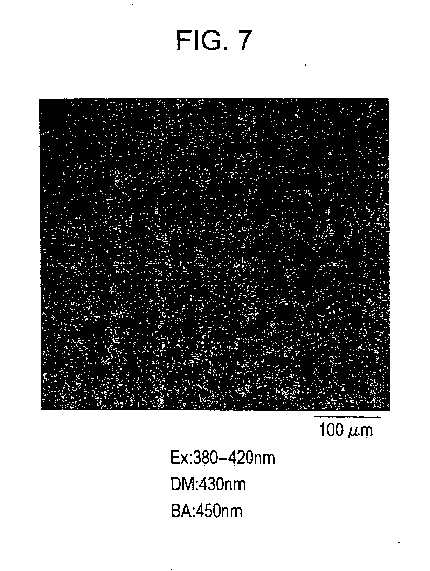

Fig. 5, Fig. 6, and Fig. 7 are fluorescent microscope photographs of ADH fluorescently labeled with cyanine dye Cy2, DI fluorescently labeled with cyanine dye Cy3, and NADH-enclosed liposome, respectively. In Fig. 5, Fig. 6, and Fig. 7, Ex (Excitation) denotes exciting light having a wavelength described on the right of Ex, and DM (Dichroic mirror) denotes a mirror for separating the exciting light and fluorescent light, which transmits only light at the wavelength described on the right of MD or more, and BA (Barrier filter) denotes a filter for separating the fluorescent light and scattering light, which transmits light at the wavelength described on the right of BA or more. In the fluorescent microscope, the dye is excited with exciting light, and the resulting light is successively passed through DM and BA to remove unnecessary light and detect only fluorescent light from the dye. Fig. 5 shows a distribution of ADH in which Cy2 was excited with the exciting light at a wavelength of 450 to 490 nm to produce fluorescent light. Fig. 6 shows a distribution of DI in which Cy3 was excited with the exciting light at a wavelength of 510 to 560 nm to produce fluorescent light. Fig. 7 shows a distribution of NADH in which NADH was excited with the exciting light at a wavelength of 380 to 420 nm to produce fluorescent light.

According to Fig. 5, Fig. 6, and Fig. 7, the average diameter of liposome was 4.6 µm, and the standard deviation was 2.0 µm. However, in Fig. 5, Fig. 6, and Fig. 7, the average diameter of liposome was determined by measuring 30 liposomes and averaging the measurements.

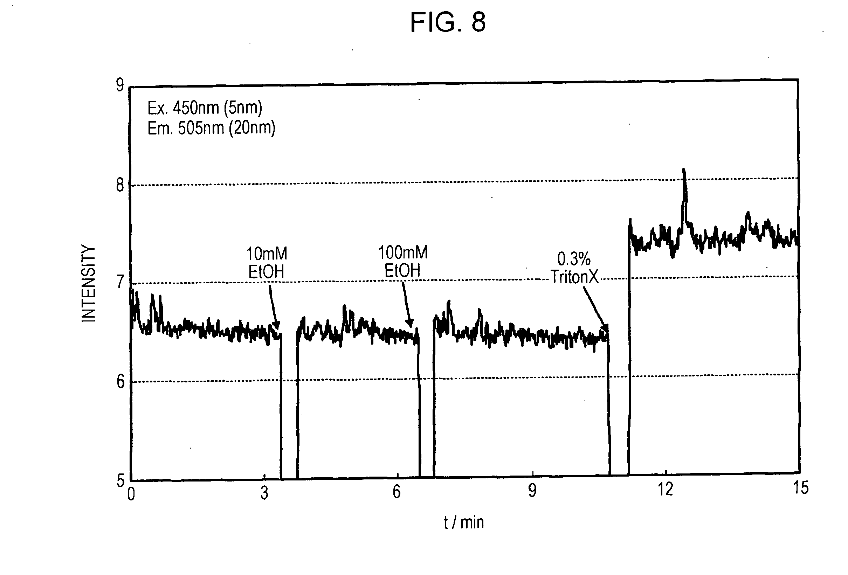

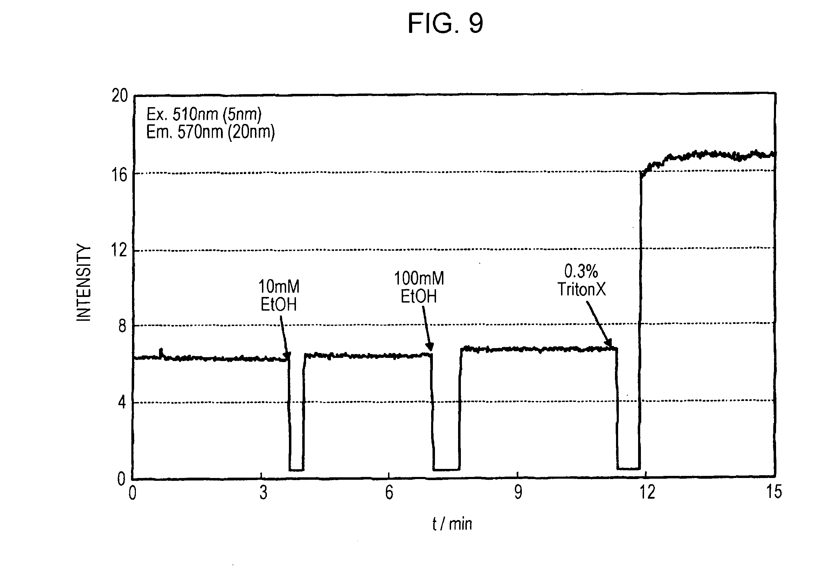

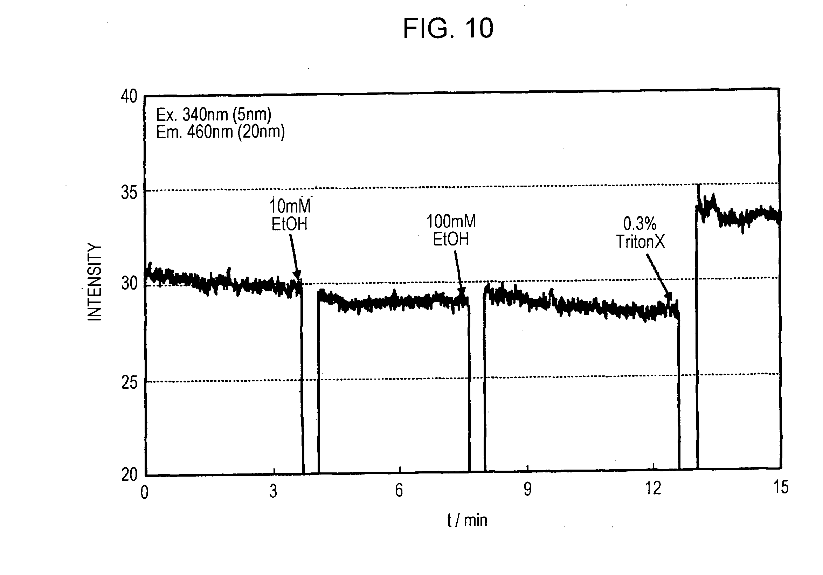

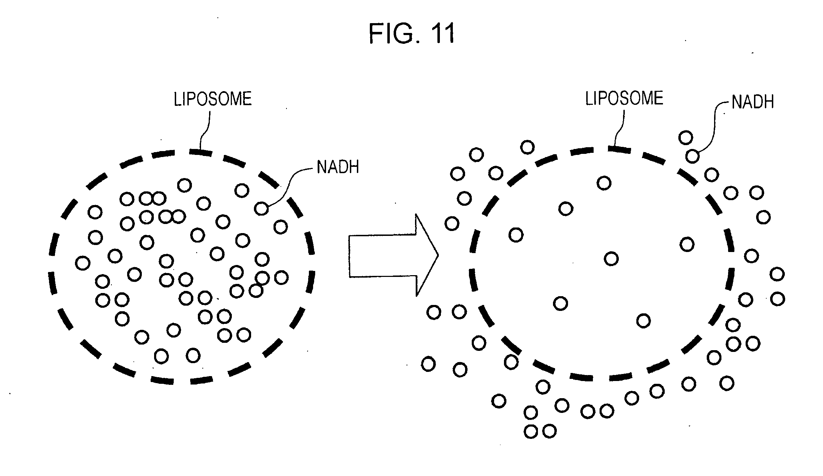

Fig. 8, Fig. 9, and Fig. 10 are graphs showing the results of fluorescent monitoring of ADH fluorescently labeled with Cy2, DI fluorescently labeled with Cy3, and NADH-enclosed liposome, respectively. Fig. 8 shows fluorescence intensity from ADH fluorescently labeled with Cy2, Fig. 6 shows fluorescence intensity from DI fluorescently labeled with Cy3, and Fig. 10 shows fluorescence intensity from NADH. In Fig. 8, Fig. 9, and Fig. 10, Em (Emission) denotes light emitted by exciting the dye with exciting light Ex, which has a wavelength described on the right of Em, and a value in parentheses on the right of the wavelength of each of Ex and Em denotes a half-value width. As seen from Fig. 8, Fig. 9, and Fig. 10, in any of the cases, the fluorescence intensity was not changed up to an ethyl alcohol concentration of 100 mM. That is, it was found that the liposome is stable up to an ethyl alcohol concentration of 100 mM, and ADH, DI, and NADH are securely enclosed in the liposome. In addition, when 0.3% Triton X, which was a surfactant, was added, the fluorescence intensity was increased. This represents that the liposome is disrupted with 0.3% Triton X, and ADH, DI, and NADH in the liposome are released to the outside of the liposome, thereby increasing the fluorescence intensity. This state is shown in Fig. 11 by taking NADH as an example. As shown in Fig. 11, under the condition in which NADH is enclosed in the liposome, the fluorescence intensity of NADH is low, while when the liposome is disrupted to release NADH enclosed in the liposome to the outside the liposome, the fluorescence intensity of NADH is increased.

The thus-formed ADH/DI/NADH-enclosed liposome solution (6) was mixed with the Q0 buffer solution (4) to prepare a total volume of 100 µL of measurement solution, and chronoamperometry was performed under stirring of the solution using a carbon felt as a working electrode at a potential set to 0.3 V with respect to a reference electrode AglAgCl, which was sufficiently higher than the oxidation-reduction potential of the electron mediator. During the chronoamperometry, ethyl alcohol was successively added so that the final concentration was 1, 10, and 100 mM.

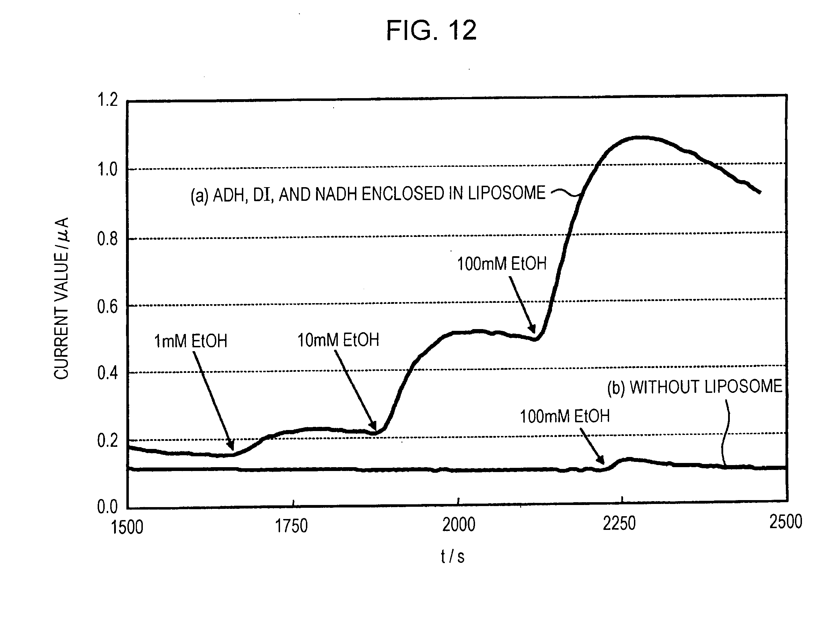

In Fig. 12, curve (a) shows the results of chronoamperometry where ethyl alcohol was added to the measurement solution containing the ADH, DI, NADH-enclosed liposome as described above. As seen from the curve (a), when ADH, DI, and NADH are enclosed in liposome, a catalyst current due to ethyl alcohol is observed, and the catalyst current increases as the concentration of ethyl alcohol increases. That is, electrochemical catalyst activity due to the ADH/DI/NADH-enclosed liposome, which is an artificial cell, was observed. On the other hand, chronoamperometry was performed in the same manner as described above for the case where ADH, DI, and NADH were simply dispersed in the Q0 buffer solution (4) without being enclosed in liposome, the amounts ADH, DI, and NADH being the same as in the case where ADH, DI, and NADH were enclosed in liposome. The results are shown by curve (b) in Fig. 12. As seen from the curve (b), when ADH, DI, and NADH were simply dispersed in the Q0 buffer solution (4) without being enclosed in liposome, substantially no catalyst current due to ethyl alcohol was observed. For example, in comparison at an ethyl alcohol concentration of 100 mM, when ADH, DI, and NADH were simply dispersed in the Q0 buffer solution (4) without being enclosed in liposome, the produced catalyst current is only about 1/30 of that of the case where ADH, DI, and NADH were enclosed in liposome. This indicates that when ADH, DI, and NADH are enclosed in liposome, an extremely high catalyst current can be obtained as compared with the case where ADH, DI, and NADH are not enclosed in liposome.

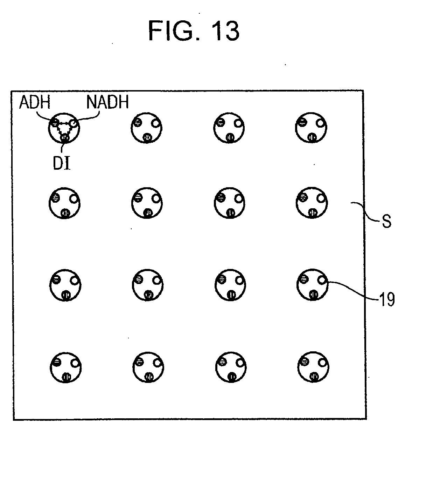

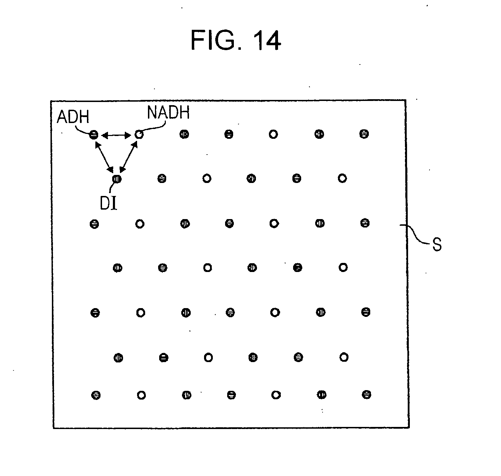

The reason why when ADH, DI, and NADH are enclosed in liposome, an extremely high catalyst current can be obtained as compared with the case where ADH, DI, and NADH are not enclosed in liposome is described. Consideration is given to the case in which liposome 19 enclosing ADH (shown by circles with horizontal lines), DI (shown by circles with vertical lines), and NADH (shown by blank circles) is arranged in a tetragonal lattice pattern in a buffer solution S as shown in Fig. 13. On the other hand, consideration is given to the case in which as shown in Fig. 14, ADH (shown by circles with horizontal lines), DI (shown by circles with vertical lines), and NADH (shown by blank circles) in the same amounts as in the case shown in Fig. 13 are arranged in a tetragonal lattice pattern in a buffer solution S in the same volume as in the case shown in Fig. 13. The volume of the buffer solution S is, for example, 100 µL, and the inner volume of the liposome 19 is, for example, about 0.17 µL. However, the phospholipid constituting the bimolecular lipid membrane of the liposome 19 is present at 5.5 × 10⁻³ µmol in the buffer solution S, and the total inner volume of the liposome 19 is 30 µL/µmol in terms of volume per µmol of the liposome 19. As shown in Fig. 14, when ADH, DI, and NADH are enclosed in the liposome 19 as shown in Fig. 13, the local concentration of these ADH, DI, and NADH is about 600 times as high as that when ADH, DI, and NADH are simply dispersed in the buffer solution S as shown in Fig. 14. Namely, the concentration of these ADH, DI, and NADH can be significantly increased by enclosing ADH, DI, and NADH in the liposome 19, and the distances between the ADH, DI, and NADH can be significantly decreased. Therefore, the catalyst cycle of the ADH, DI, and NADH can be allowed to proceed at a high rate in the liposome 19, thereby achieving the results as shown in Fig. 12.

It is found from the above that in the first embodiment, when the enzymes 13 and 14 and the coenzyme 15 necessary for the enzyme reaction are enclosed in the liposome 12, the enzyme reaction can be efficiently effected using a micro space in the liposome as a reaction field, and electrons can be efficiently extracted from the substrate.

### (EXAMPLE 1)

An enzyme-immobilized electrode was formed as follows.

One to ten mg of diaphorase (DI) (EC 1.8.1.4, manufactured by Amano Enzyme Inc.) was weighed and dissolved in 1 mL of a buffer solution (10 mM phosphate buffer solution, pH 7) to prepare a DI enzyme buffer solution (11).

Ten to fifty mg of glucose dehydrogenase (GDH) (NAD-dependent, EC 1.1.1.47, manufactured by Amano Enzyme Inc.) was weighed and dissolved in 1 mL of a buffer solution (10 mM phosphate buffer solution, pH 7) to prepare a GDH enzyme buffer solution (12).

The buffer solution in which each of the enzymes was dissolved is preferably refrigerated until just before dissolving, and the enzyme buffer solutions are also preferably refrigerated as far as possible.

Ten to fifty mg of NADH (manufactured by Sigma-Aldrich Ltd., N-8129) was weighed and dissolved in 1 mL of a buffer solution (10 mM phosphate buffer solution, pH 7) to prepare a NADH enzyme buffer solution (13).

One hundred to two hundred mg of 2,3-dimethoxy-5-methyl-1,4-benzoquinone (Q0) was weighed and dissolved in 1 mL of a buffer solution (10 mM phosphate buffer solution, pH 7) to prepare a Q0 buffer solution (14).

Ten to one hundred mg of yolk lecithin (manufactured by Wako) was weighed and dissolved in 10 mL of a buffer solution (10 mM phosphate buffer solution, pH 7) and homogenized with a homogenizer to prepare a liposome buffer solution (15).

The amount described below was sampled from each of the solutions prepared as described above, and the samples were mixed, followed by repetition of three times of freeze thawing.
DI enzyme buffer solution (11): 50 µL
GDH enzyme buffer solution (12): 50 µL
NADH buffer solution (13): 50 µL
Liposome buffer solution (15): 50 µL

The above-described mixed solution was passed through a gel filtration column to remove the enzymes and NADH outside the liposome. The resultant liposome solution was regarded as a GDH/DI/NADH-enclosed liposome solution (16).

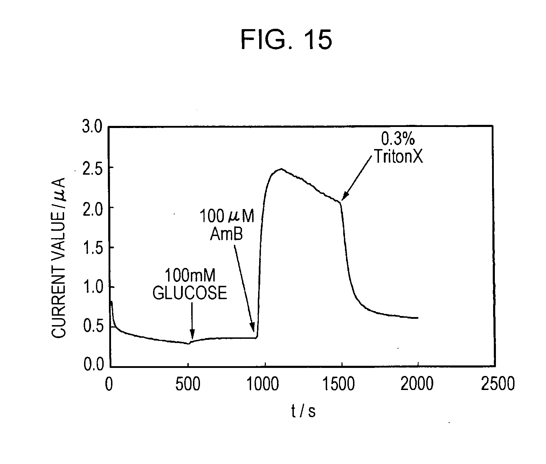

The thus-formed GDH/DI/NADH-enclosed liposome solution (16) was mixed with the Q0 buffer solution (14) to prepare a total volume of 100 µL of measurement solution. Then, chronoamperometry was performed under stirring of the solution using glucose as a fuel, a carbon felt as a working electrode, AglAgCl as a reference electrode, and a platinum wire as a counter electrode at a potential set to +0.1 V with respect to the AglAgCl. The results of chronoamperometry are shown in Fig. 15.

As shown in Fig. 15, first, when glucose was added to the measurement solution during chronoamperometry so that the final concentration was 100 mM, substantially no reaction occurred, and the obtained current value was little changed. This is due to the fact that glucose is permeated through the bimolecular lipid membrane constituting the liposome at an extremely low rate and substantially not permeated.

Next, amphotericin B (AmB) was added as the antibiotic 16 to the measurement solution so that the final concentration was 100 µM. As a result, a catalyst current due to glucose was observed. Namely, it was found that glucose is permeated through the bimolecular lipid membrane constituting the liposome, thereby causing a series of enzyme reaction to proceed in the liposome. As far as the inventors known, a catalyst current due to glucose enclosed in liposome as described above was observed for the first time.

Next, when 0.3% Triton X serving as a surfactant which can disrupt liposome was added to the measurement solution, the catalyst current was significantly decreased. This indicates that the liposome is disrupted with 0.3% Triton X, thereby releasing GDH, DI, and NADH to the outside of liposome.

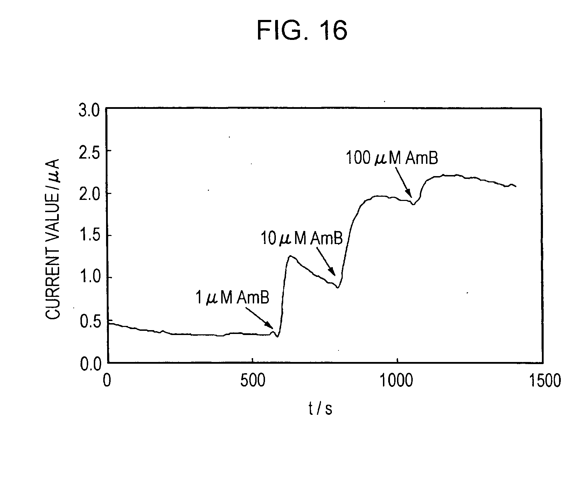

Fig. 16 shows the results of chronoamperometry performed by changing, to 1, 10, and 100 µM, the final concentration of amphotericin B added to the same measurement solution containing 100 mM of glucose as described above. As shown in Fig. 16, the catalyst current increases as the final concentration of amphotericin B added increases within the concentration range of 1 to 100 µM, but the catalyst current shows a tendency to reach the top of increase at the final concentration of amphotericin B of 100 µM.

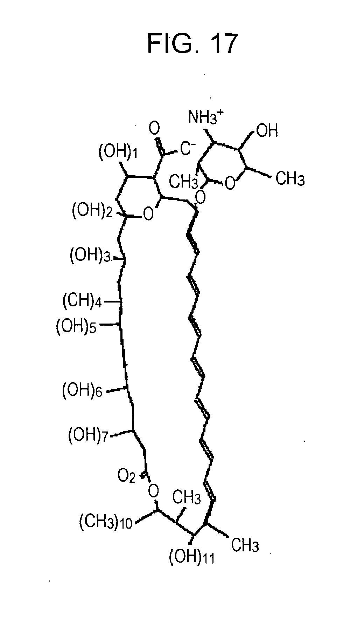

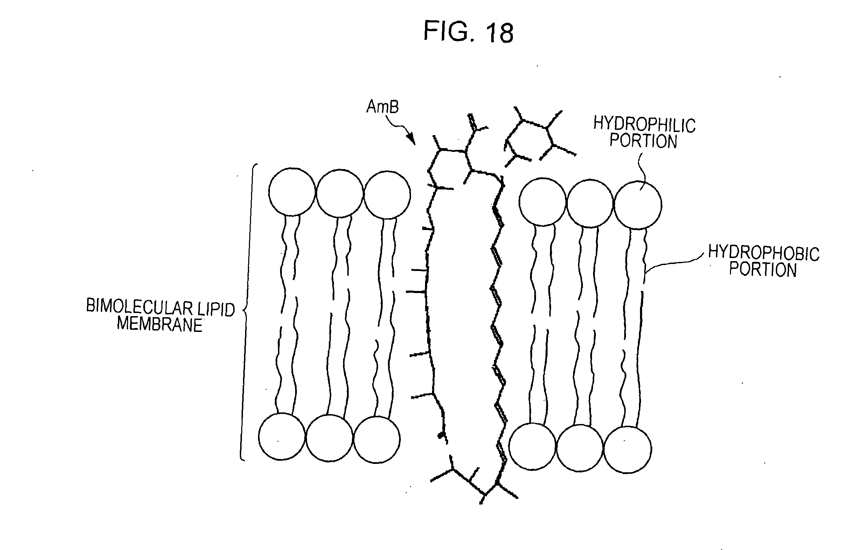

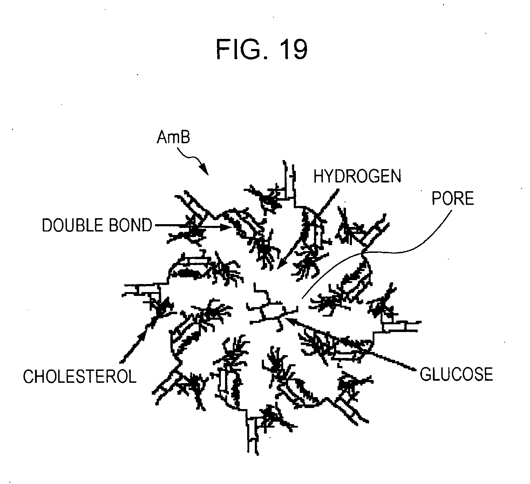

Fig. 17 shows a structural formula of amphotericin B. In addition, Fig. 18 shows a sectional view showing a state in which amphotericin B is bonded to the bimolecular lipid membrane constituting the liposome 12. Further, Fig. 19 shows a plan view showing a state in which amphotericin B is bonded to the bimolecular lipid membrane (not shown). As shown in Fig. 19, a plurality of molecules of amphotericin B bonded in a ring form to the bimolecular lipid membrane form a pore edged with the amphotericin B and passing through the bimolecular lipid membrane. It has been reported that a pore can be formed in liposome by amphotericin B (refer to, for example, Non-Patent Literature 3). The pore has a size such that glucose can be permeated, while the enzymes GDH and DI and the coenzyme NADH are not permeated.

As described above, according to the first embodiment, the enzymes 13 and 14 and the coenzyme 15 necessary for the enzyme reaction for decomposing glucose are enclosed in the liposome 12 in which the pore 17 is formed by bonding the antibiotic 16 to the bimolecular lipid membrane. In addition, the liposome 12 is immobilized on the electrode 11. Therefore, the enzyme reaction can be efficiently effected using as a reaction field a micro space in the liposome 12, and electrons can be efficiently extracted from the glucose serving as the substrate and transferred to the electrode 11. In addition, immobilization can be simply performed as compared with the case of conventional direct immobilization of an enzyme and the like on the electrode 11 by a polyion complex or the like.

### (2. Second Embodiment)

### [Biofuel cell]

Next, a second embodiment of the present invention is described. In the second embodiment, the enzyme-immobilized electrode according to the first embodiment is used as a negative electrode of a biofuel cell.

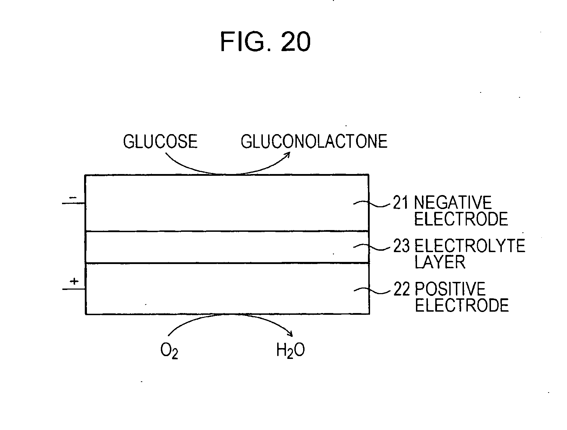

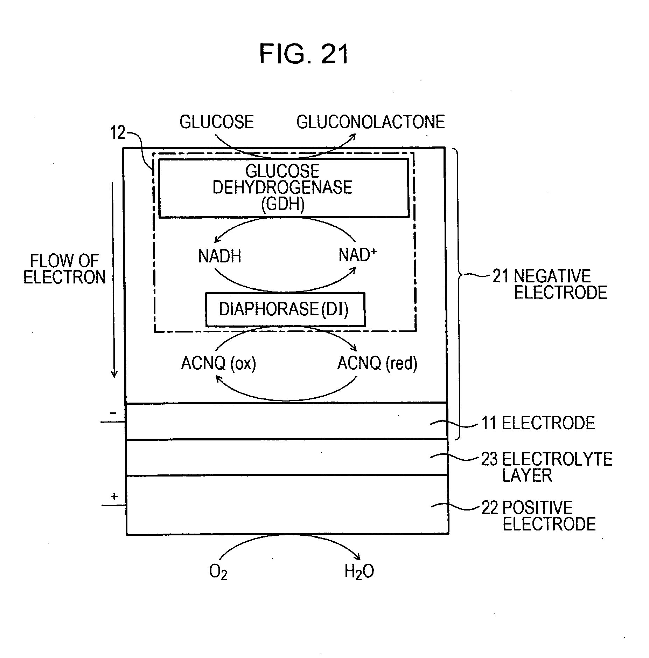

Fig. 20 schematically shows the biofuel cell. The biofuel cell uses glucose as fuel. Fig. 21 schematically shows a detailed configuration of the negative electrode of the biofuel cell, examples of an enzyme group and a coenzyme enclosed in the liposome 12 immobilized on the negative electrode, and an electron transfer reaction of the enzyme group and the coenzyme.

As shown in Fig. 20 and Fig. 21, the biofuel cell has a structure in which a negative electrode 21 and a positive electrode 22 face each other with an electrolyte layer 23 provided therebetween. On the negative electrode 21, the glucose supplied as the fuel is decomposed with an enzyme to extract electrons and produce protons (H⁺). On the positive electrode 22, water is produced from the protons transferred from the negative electrode 21 through the electrolyte layer 23, electrons transferred from the negative electrode 21 through an external circuit, and oxygen in air.

As the negative electrode 21, the enzyme-immobilized electrode according to the first embodiment is used. Specifically, the enzyme-immobilized electrode includes the electrode 11 on which the liposome 12 having the pore 17 permeable to glucose and formed in the bimolecular lipid membrane is immobilized. In the liposome 12, an enzymes involved in decomposition of glucose, a coenzyme which produces a reduced form with an oxidation reaction in the glucose decomposition process, and a coenzyme oxidase which oxidizes the reduced form of the coenzyme are enclosed. If required, in addition to the liposome 12, an electron mediator which receives electrons produced with the oxidation of the coenzyme from the coenzyme oxidase and transfers the electrons to the electrode 11 is also immobilized on the electrode 11.

As the enzyme involved in decomposition of glucose, for example, glucose dehydrogenase (GDH), preferably NAD-dependent glucose dehydrogenase, can be used. In the presence of the oxidase, for example, β-D-glucose can be oxidized to D-glucono-δ-lactone.

Further, this D-glucono-δ-lactone can be decomposed into 2-keto-6-phospho-D-gluconate in the presence of the two enzymes of gluconokinase and phosphogluconate dehydrogenase (PhGDH). That is, the D-glucono-δ-lactone is converted to D-gluconate by hydrolysis, and D-gluconate is phosphorylated to 6-phospho-D-gluconate by hydrolyzing adenosine triphosphate (ATP) into adenosine diphosphate (ADP) and phosphoric acid in the presence of gluconokinase. This 6-phospho-D-gluconate is oxidized to 2-keto-6-phospho-D-gluconate by the action of oxidase PhGDH.

In addition, besides the above-described decomposition process, glucose can be decomposed to CO₂ by using sugar metabolism. The decomposition process using sugar metabolism is roughly divided into decomposition of glucose by a glycolysis system, formation of pyruvic acid, and TCA cycle, but these are well known reaction systems.

An oxidation reaction in a decomposition process of a monosaccharide is performed accompanying a reduction reaction of a coenzyme. The coenzyme is substantially determined depending on the enzyme acting, and in the case of GDH, NAD⁺ is used as the coenzyme. Namely, when β-D-glucose is oxidized to D-glucono-δ-lactone by the action of GDH, NAD⁺ is reduced to NADH to produce H⁺.

The produced NADH is immediately oxidized to NAD⁺ in the presence of diaphorase (DI) to produce two electrons and H⁺. Therefore, two electrons and two H⁺ are produced by one step of oxidation reaction per molecule of glucose. In two steps of oxidation reaction, four electrons and four H⁺ in total are produced.

The electrons produced in the above-described process are transferred to the electrode 11 from diaphorase through the electron mediator, and H⁺ are transported to the positive electrode 22 through the electrolyte layer 23.

The liposome 12 in which the enzyme and the coenzyme are enclosed, and the electron mediator are preferably maintained at optimum pH for the enzyme, for example, close to pH 7, with a buffer solution such as a phosphate buffer solution, a tris buffer solution, or the like, which is contained in the electrolyte layer 23, in order to permit efficient and stationary electrode reaction. As the phosphate buffer solution, for example, NaH₂PO₄ or KH₂PO₄ is used. Further, excessively high or low ionic strength (I. S.) adversely affects enzyme activity, but proper ionic strength, for example, about 0.3, is preferable in view of electrochemical response. However, there are optimum values of pH and ionic strength for each of the enzymes used, and the pH and ionic strength are not limited to the above values.

Fig. 21 illustrates, as an example, a case where the enzyme involved in decomposition of glucose is glucose dehydrogenase (GDH), the coenzyme which produces a reduced form with oxidation reaction in the decomposition process of glucose is NAD⁺, the coenzyme oxidase which oxidizes NADH as a reduced form of the coenzyme is diaphorase (DI), and the electron mediator which receives electrons produced with oxidation of the coenzyme from the coenzyme oxidase and transfers the electrons to the electrode 11 is ACNQ.

The positive electrode 22 includes an electrode composed of an electrode material, for example, porous carbon or the like, and an enzyme which decomposes oxygen, for example, bilirubin oxidase, laccase, ascorbate oxidase, or the like, the enzyme being immobilized on the electrode. An outer portion (a portion opposite to the electrolyte layer 23) of the positive electrode 22 is usually made of a gas diffusion layer composed of porous carbon, but the outer portion is not limited to this. In addition to the enzyme, preferably, an electron mediator which transfers electrons to the positive electrode 22 is also immobilized on the positive electrode 22.

On the positive electrode 22, oxygen in air is reduced in the presence of the enzyme which decomposes oxygen, producing water due to H⁺ from the electrolyte layer 23 and the electrons from the negative electrode 21.

The electrolyte layer 23 is adapted for transporting the H⁺ produced on the negative electrode 21 to the positive electrode 22 and is composed of a material which has no electron conductivity and which can transport H⁺. As the electrolyte layer 23, specifically, for example, the above-described material such as cellophane or the like can be used.

In the biofuel cell configured as described above, when glucose is supplied to the negative electrode 21, the glucose is decomposed with a catabolic enzyme including oxidase. Since the oxidase is involved in the decomposition process of a monosaccharide, electrons and H⁺ can be produced on the negative electrode 21 side, and a current can be produced between the negative electrode 21 and the positive electrode 22.

Next, a specific example of a structure of the biofuel cell is described.

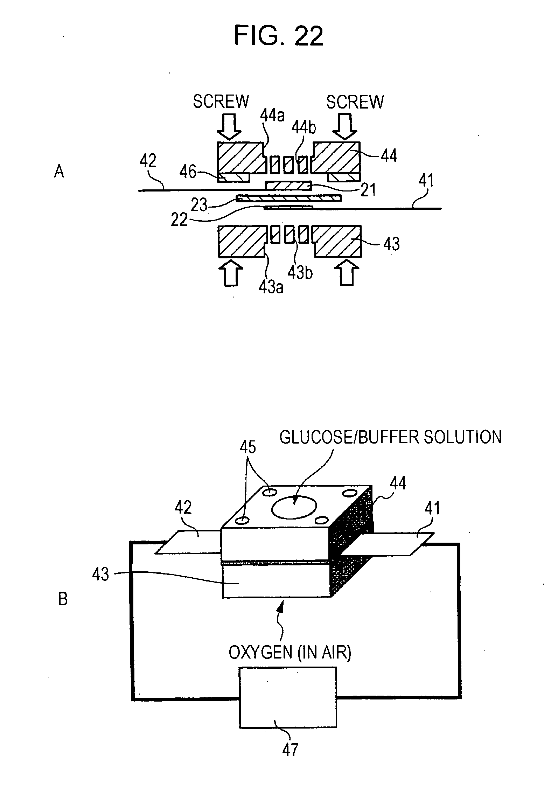

As shown in Figs. 22A and B, the biofuel cell has a configuration in which the negative electrode 21 and the positive electrode 22 face each other with the electrolyte layer 23 provided therebetween. In this case, Ti current collectors 41 and 42 are disposed below the positive electrode 22 and the negative electrode 21, respectively, so that a current can be easily collected. Reference numerals 43 and 44 each denote a fixing plate. The fixing plates 43 and 44 are fastened with screws 45 to hold all the positive electrode 22, the negative electrode 21, the electrolyte layer 23, and the Ti current collectors 41 and 42 therebetween. An air-intake circular recess 43a is provided in one (outer side) of the sides of the fixing plate 43, and many holes 43b are provided at the bottom of the recess 43a so as to pass to the other side. These holes 43b serve as an air supply passage to the positive electrode 22. On the other hand, a fuel charging circular recess 44a is provided in one (outer side) of the sides of the fixing plate 44, and many holes 44b are provided at the bottom of the recess 44a so as to pass to the other side. These holes 44b serve as an fuel supply passage to the negative electrode 21. A spacer 46 is provided in the peripheral portion of the other side of the fixing plate 44 so as to form a predetermined space between the fixing plates 43 and 44 when the fixing plates 43 and 44 are fastened together with the screws 45.

As shown in Fig. 22B, a load 47 is connected between the Ti current collectors 41 and 42 so that electric power is generated by placing, as the fuel, a glucose solution which contains glucose dissolved in a phosphate buffer solution in the recess 44a of the fixing plate 44.

According to the second embodiment, the enzyme-immobilized electrode including the electrode 11 on which the liposome 12 having the pore 17, which is formed in the bimolecular lipid membrane by bonding the antibiotic 16, is immobilized is used as the negative electrode 21, the enzyme group and the coenzyme necessary for the enzyme reaction being enclosed in the liposome 12. Therefore, the enzyme reaction can be efficiently effected using the micro space in the liposome 12 as a reaction field, electrons can be efficiently extracted from the glucose as the fuel and transferred to the electrode 11, and the enzyme and the like can be simply immobilized as compared with direct immobilization on the electrode 11 using a polyion complex or the like. Since the enzyme-immobilized electrode used as the negative electrode 21 has high efficiency, a high-efficiency biofuel cell can be realized. In addition, in order to realize higher output of a biofuel cell, it is necessary to extract two or more electrons from glucose used as the fuel, thereby causing the need to use as the negative electrode 21 an enzyme-immobilized electrode on which three or more types of enzymes are immobilized at proper positions. However, this requirement can also be satisfied by enclosing three or more types of enzymes in the liposome 12. In addition, application to various types of fuels can be easily made by mixing many types of liposomes in which three or more different types of enzymes are respectively enclosed. Further, a micro-biofuel cell in which negative electrode liposome and positive electrode liposome are arranged can also be realized.

### (3. Third embodiment)

### [Biofuel cell]

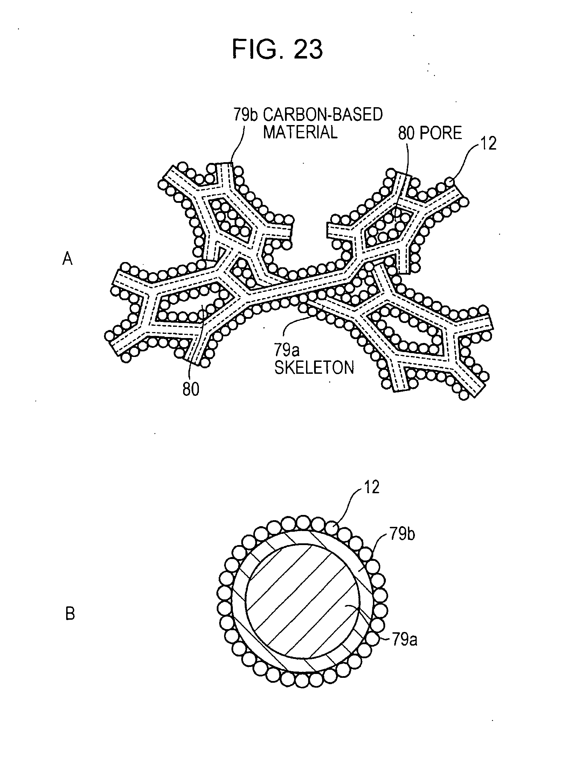

A biofuel cell according to a third embodiment has the same configuration as the biofuel cell according to the second embodiment except that a porous conductive material as shown in Figs. 23A and B is used as an electrode material of the negative electrode 21.

Fig. 23A schematically shows a structure of the porous conductive material, and Fig. 23B is a sectional view of a skeleton portion of the porous conductive material. As shown in Figs. 23A and B, the porous conductive material includes a skeleton 79a composed of a porous material with a three-dimensional network structure and a carbon-based material 79b which covers the surface of the skeleton 79a, the same liposome 12 as in the second embodiment being immobilized on the surface of the carbon-based material 79b. The porous conductive material has a three-dimensional network structure in which many pores 80 surrounded by the carbon-based material 79b correspond to meshes. In this case, the pores 80 communicate with each other. The form of the carbon-based material 79b is no object and may be any one of a fibrous form (needle-like), a granular form, and the like.

As the skeleton 79a composed of the porous material, a foam metal or a foam alloy, for example, foam nickel, can be used. The porosity of the skeleton 79a is generally 85% or more or 90% or more, and the pore size is generally, for example, 10 nm to 1 mm, 10 nm to 600 µm, or 1 to 600 µm, typically 50 to 300 µm, more typically 100 to 250 µm. As the carbon-based material 79b, for example, a high-conductivity material such as Ketjenblack or the like is preferred, but a functional carbon material such as carbon nanotubes, fullerene, or the like may be used.

The porosity of the porous conductive material is generally 80% or more or 90% or more, and the diameter of the pores 80 is generally, for example, 9 nm to 1 mm, 9 nm to 600 µm, or 1 to 600 µm, typically 30 to 400 µm, more typically 80 to 230 µm.

Next, a method for producing the porous conductive material is described.

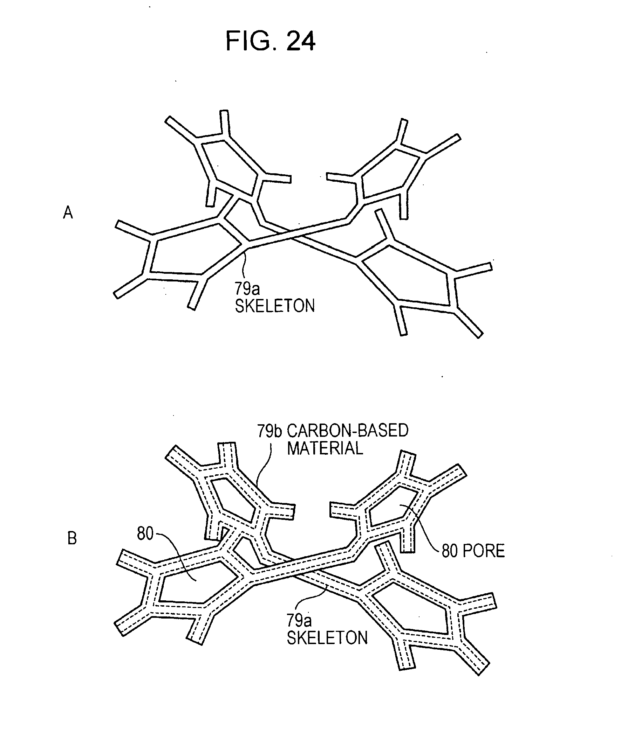

As shown in Fig. 24A, first the skeleton 79a composed of a foam metal or a form alloy (for example, foam nickel) is prepared.

Next, as shown in Fig. 24B, the surface of the skeleton 79a composed of a foam metal or a foam alloy is coated with the carbon-based material 79b. As the coating method, a conventional know method can be used. For example, the surface of the skeleton 79a is coated with the carbon-based material 79b by spraying an emulsion containing a carbon powder, a proper binder, and the like using a spray. The coating thickness of the carbon-based material 79b is determined according to the porosity and pore diameter required for the porous conductive material with the balance with the porosity and pore diameter of the skeleton 79a composed of a foam metal or a foam alloy. During coating, the many pores 80 surrounded by the carbon-based material 79b are communicated with each other.

As a result, the intended porous conductive material is produced. Then, the liposome 12 is immobilized on the surface of the carbon-based material 79b of the porous conductive material.

Conditions other than the above are the same as in the second embodiment.

According to the third embodiment, in addition to the advantages of the second embodiment, the advantages described below can be obtained. Namely, the porous conductive material including the skeleton 79a composed of a foam metal or a foam alloy and having the surface coated with the carbon-based material 79b has the pores 80 with a sufficiently large diameter and has high strength and high conductivity while maintaining a coarse three-dimensional network structure, and a necessary and sufficient surface area can be obtained. Therefore, the negative electrode 21 including an enzyme/coenzyme/electron mediator-immobilized electrode which is produced by immobilizing an enzyme, a coenzyme, and an electron mediator on the porous conductive material used as an electrode material is capable of effecting high-efficiency enzyme metabolic reaction thereon and efficiently capturing, as an electric signal, an enzyme reaction phenomenon occurring near the electrode, and is stable regardless of the use environment, and a high-performance biofuel cell can be realized.

### (4. Fourth embodiment)

### [Cells]

In the fourth embodiment, one or more pores permeable to glucose are formed in a bimolecular lipid membrane constituting a cell membrane of cells collected from an organism or cells obtained by culturing cells collected from an organism.

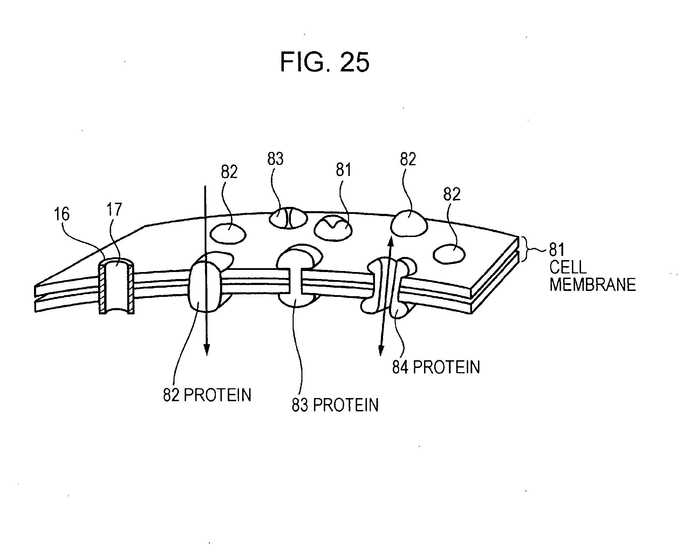

Fig. 25 shows a portion of a cell membrane 81 composed of a bimolecular lipid membrane in the periphery of a cell. Although, herein, a case where the cell is an animal cell is considered, the cell may be a plant cell. The cell mainly contains a nucleus, cell organelles such as a mitochondrion, an endoplasmic reticulum, a Golgi body, lysosome, and the like, and water in which various ions, nutrients, enzymes, and the like are dissolved. As shown in Fig. 25, various proteins 82, 83, and 84 are buried in the cell membrane 81. For example, the protein 82 is a pump protein which transports ions and the like from the outside to the inside of the cell, the protein 83 is a membrane integral protein, and the protein 84 is a channel protein having the function to transport specified molecules between the inside and outside of the cell. In addition to the above various proteins 82, 83, and 84, an antibiotic 16 is bonded to the cell membrane 81 to form one or more pores 17 permeable to glucose.

The cells configured as described are immobilized on an electrode composed of porous carbon or the like by a conventional known method.

According to the fourth embodiment, when the cells are placed in a buffer solution containing glucose, glucose can be entrapped by permeation through the pores 17 of the cell membrane 81. The glucose entrapped in the cells is decomposed by a metabolic system such as a citric acid cycle, a glycolysis system, a pentose phosphate cycle, or the like to release electrons. The released electrons are finally transferred to the electrode on which the cells are immobilized through the electron mediator or directly without the electron mediator, and emitted to the outside. Consequently, electric energy can be generated from glucose.

### (5. Fifth embodiment)

### [Mitochondria]

In the fifth embodiment, one or more pores permeable to glucose are formed in a bimolecular lipid membrane constituting mitochondria as cell organelles contained in cells collected from an organism or cells obtained by culturing cells collected from an organism. The mitochondria serve as energy production fields of eucaryotic cells and produce ATP with energy obtained by molecular oxidation reaction of food.

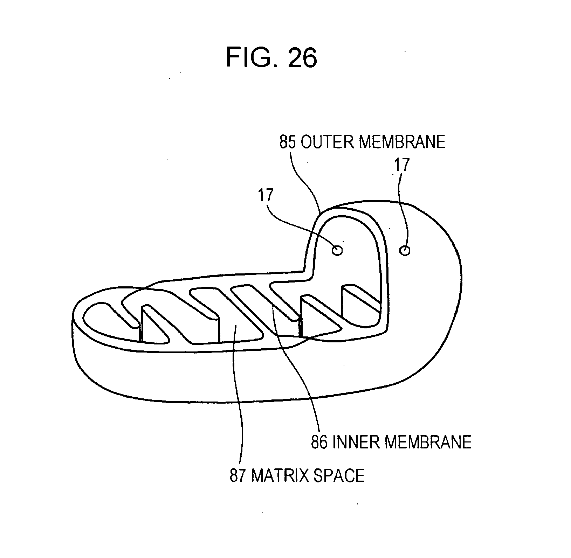

Fig. 26 shows a mitochondrion. As shown in Fig. 26, the mitochondrion has an outer membrane 85 and an inner membrane 86. These outer membrane 85 and inner membrane 86 are cell membranes each including a bimolecular lipid membrane. The inner membrane 86 forms a folded crista. Many types of enzymes are concentrated in a matrix space 87 inside the inner membrane 86. The final stage of molecular oxidation occurs in the inner membrane 86. The antibiotic 16 is bonded to the cell membrane constituting the outer membrane 85 and the inner membrane 86 of the mitochondrion to form one or more pores 17 permeable to glucose.

The mitochondria configured as described above are immobilized on an electrode composed of porous carbon by a conventional known method.

According to the fifth embodiment, when the mitochondria are placed in a buffer solution containing glucose, glucose can be entrapped by permeation through the pores 17 of the outer membrane 85 and the inner membrane 86. The glucose entrapped in the mitochondria is decomposed by an oxidation system possessed by the mitochondria, such as a citric acid cycle, a glycolysis system, a pentose phosphate cycle, or the like to release electrons. The released electrons are finally transferred to the electrode on which the mitochondria are immobilized through the electron mediator or directly without the electron mediator, and emitted to the outside. Consequently, electric energy can be generated from glucose.

### (6. Sixth embodiment)

### [Bacteria]

In the sixth embodiment, one or more pores permeable to glucose are formed in a bimolecular lipid membrane constituting a cell membrane of a bacterium.

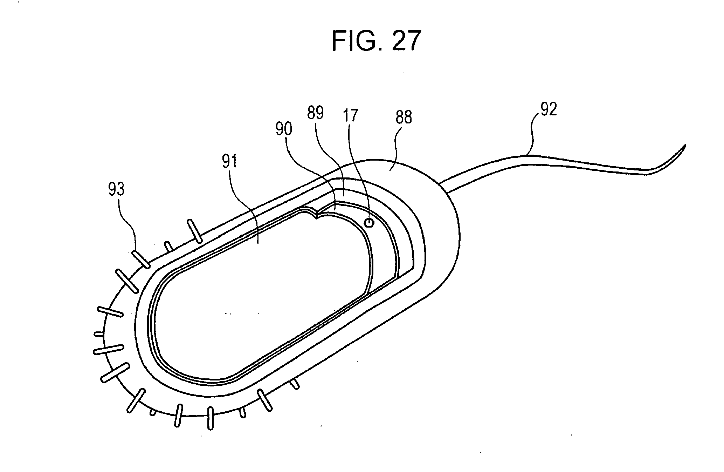

Fig. 27 shows a bacterium. Here, a case wherein the bacterium is an intrinsic cell is considered. As shown in Fig. 27, the bacterium is surround by a capsule 88, a cell wall 89, and a cell membrane 90 in that order from the outside, and contains cytoplasma 91 therein. A flagellum 92 and a cilia 93 are bonded to the outside of the bacterium. However, some types of bacteria do not have the capsule 88, the flagellum 92, and the cilia 93. For example, Escherichia coli does not have the capsule 88, the flagellum 92, and the cilia 93. Although not shown in the drawing, a nucleoid, ribosome, and the like are present in the cytoplasma 91. In the cytoplasma 91, an electron transfer system and proteins such as various transporters are distributed in the cell membrane 90. An antibiotic 16 is bonded to the cell membrane 90 including a bimolecular lipid membrane to form one or more pores 17 permeable to glucose.

The bacteria configured as described above are immobilized on an electrode composed of porous carbon by a conventional known method.

According to the sixth embodiment, when the bacteria are placed in a buffer solution containing glucose, glucose can be entrapped by permeation through the pores 17 of the cell membrane 90 of the bacteria. The glucose entrapped in the bacteria is decomposed by a metabolic system possessed by the bacterium, such as a citric acid cycle, a glycolysis system, a pentose phosphate cycle, or the like to release electrons. The released electrons are finally transferred to the electrode on which the bacteria are immobilized through the electron mediator or directly without the electron mediator, and emitted to the outside. Consequently, electric energy can be generated from glucose.

### (7. Seventh embodiment)

### [Biofuel cell]

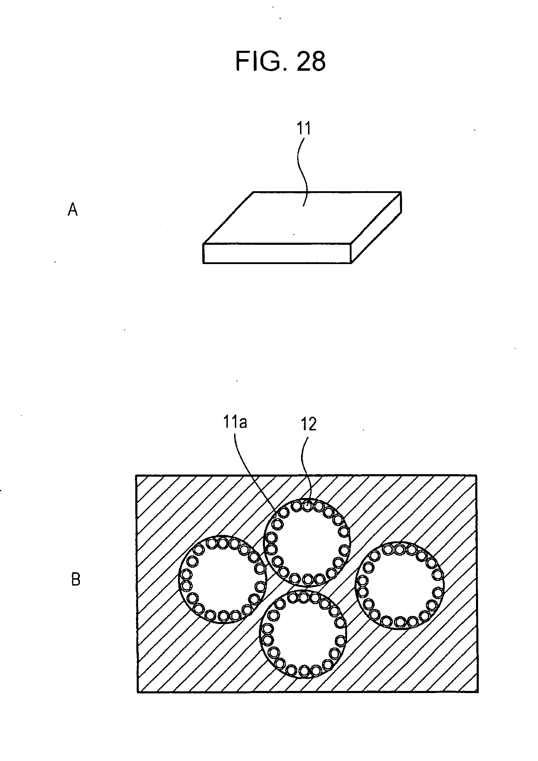

A biofuel cell according to the seventh embodiment has the same configuration as the biofuel cell according to the second embodiment except that a porous electrode as shown in Figs. 28A and B is used as the electrode 11 of the negative electrode 21.

Fig. 28A shows the whole configuration of the electrode 11 including the porous electrode, and Fig. 28B schematically shows a sectional structure of the electrode 11. As shown in Fig. 28B, the electrode 11 including the porous electrode has many pores 11a. The same liposome 12 as in the second embodiment (an enzyme and a coenzyme enclosed in an inner aqueous phase, an antibiotic bonded to the bimolecular lipid membrane, and the pores edged with the antibiotic are not shown in the drawing) is immobilized on the inner surfaces of the pores 11a. In this case, the pores 11a communicate with each other. As the material of the porous electrode, a carbon-based material is preferably used, but the material is not limited to this. The porous electrode is typically a carbon paste electrode.

The liposome 12 can be immobilized on the inner surfaces of the pores 11a of the electrode 11 including the porous electrode by permeating a solution containing the liposome 12 into the electrode 11.

Conditions other than the above are the same as in the second embodiment.

According to the seventh embodiment, the same advantages as in the third embodiment can be obtained.

### (8. Eighth embodiment)

### [Enzyme-immobilized electrode]

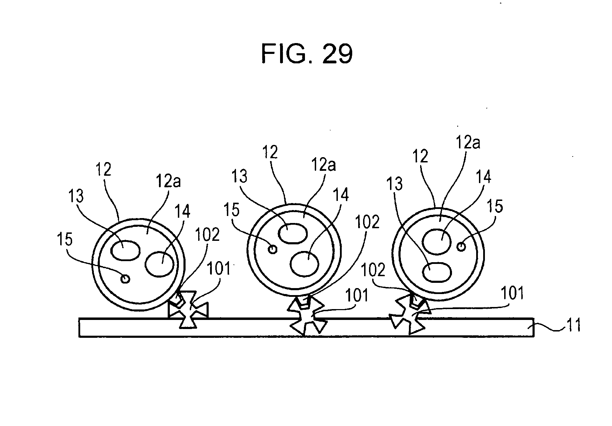

Fig. 29 shows an enzyme-immobilized electrode according to the eighth embodiment of the preset invention. In the enzyme-immobilized electrode, glucose is used as a substrate.

As shown in Fig. 29, in the enzyme-immobilized electrode, avidin 101 is immobilized on a surface (including the inner surfaces of pores in an electrode 11) of an electrode 11 composed of porous carbon or the like. On the other hand, biotin 102 is bonded to the same liposome 12 as in the first embodiment. In addition, the avidin 101 immobilized on the surface of the electrode 11 is irreversibly specifically bonded to the biotin 102 bonded to the liposome 12. Although Fig. 29 shows a case where one biotin 102 is bonded to one liposome 12, a plurality of molecules of biotin 102 may be bonded to one liposome 12.

Enzymes 13 and 14 and a coenzyme 15 involved in a target enzyme reaction are enclosed in the aqueous phase 12a in the liposome 12. Besides, theses enzymes 13 and 14 and coenzyme 15, for example, an electron mediator may be enclosed in the aqueous phase 12a in the liposome 12. The electron mediator may be immobilized on the electrode 11 together with the liposome 12. The enzyme 13 is an oxidase which promotes oxidation of glucose used as a substrate to decompose the glucose, and the enzyme 14 is a coenzyme oxidase which returns the coenzyme 15 reduced with oxidation of the glucose to an oxidized form and transfers electrons to the electrode 11 through the electron mediator.

Although not shown in the drawing, like in the liposome 12 shown in Fig. 2, one or more antibiotics 16 are bonded to the bimolecular lipid membrane constituting the liposome 12, forming pores which pass through the bimolecular lipid membrane in a form of being edged with the antibiotics 16.

### [Method for producing enzyme-immobilized electrode]

The enzyme-immobilized electrode can be produced, for example, as follows. First, the liposome 12 is formed, in which the enzymes 13 and 14 and the coenzyme 15 are enclosed and in which the biotin 102 is bonded to the periphery. In order to bond the biotin 102 to the liposome 12, for example, the biotin 102 modified with NHS ester is used and dehydration-fused with, for example, phosphatidyl ethanolamine to form an amide bond. Next, the antibiotic is bonded to the bimolecular lipid membrane constituting the liposome 12, forming the pore. On the other hand, the avidin 101 is immobilized on the surface of the electrode 11. In order to immobilize the avidin 101 on the electrode 11, for example, a carbon paste electrode mixed with agarose beads to which the avidin 101 is bonded is used as the electrode 11, or the avidin 101 is buried in a polymer such as poly-L-lycine (PLL) or the like on the surface of the electrode 11. Next, the liposome 12 is immobilized on the electrode 11 by bonding the avidin 101 immobilized on the surface of the electrode 11 and the biotin 102 bonded to the liposome 12 having the pore 17 formed therein. Consequently, the enzyme-immobilized electrode is produced. The antibiotic may be bonded to the bimolecular lipid membrane constituting the liposome 12 to form the pore before the enzymes 13 and 14 and the coenzyme 15 are enclosed. In addition, the antibiotic may be bonded to the bimolecular lipid membrane constituting the liposome 12 to form the pore after the liposome 12 is immobilized on the electrode 11.

### (EXAMPLE 2)

An enzyme-immobilized electrode was formed as follows.

Five mg of diaphorase (DI) (EC 1.8.1.4, manufactured by Amano Enzyme Inc.) was weighed and dissolved in 1 mL of a buffer solution (10 mM phosphate buffer solution, pH 7) to prepare a 5 mg/mL DI enzyme buffer solution.

Twenty-five mg of glucose dehydrogenase (GDH) (NAD-dependent, EC 1.1.1.47, manufactured by Amano Enzyme Inc.) was weighed and dissolved in 1 mL of a buffer solution (10 mM phosphate buffer solution, pH 7) to prepare a 25 mg/mL GDH enzyme buffer solution.

The buffer solution in which each of the enzymes was dissolved is preferably refrigerated until just before dissolving, and the enzyme buffer solutions are also preferably refrigerated as far as possible.

NADH (manufactured by Sigma-Aldrich Ltd.) was dissolved in a buffer solution (10 mM phosphate buffer solution, pH 7) to prepare a 50 mM NADH enzyme buffer solution.

2,3-dimethoxy-5-methyl-1,4-benzoquinone (Q0) was dissolved in a buffer solution (10 mM phosphate buffer solution, pH 7) to prepare a 10 mM Q0 buffer solution.

Yolk lecithin (manufactured by Wako) and biotin-modified phosphatidyl ethanolamine (1,2-dipalmitoyl-sn-glycero-3-phosphoethanolamine-N-(cap biotinyl) were used as phospholipids.

The biotin-modified liposome was formed as follows:

Lecithin was dissolved in ethanol to prepare a 100 mg/mL lecithin solution. In addition, biotin-modified phosphatidylethanolamine was dissolved in a mixed solution of CHCl₃, methanol, and water (CHCl₃:methanol:water = 65:35:8) to prepare a 10 mg/mL biotin-modified phosphatidylethanolamine solution.

To 20 mL of ethanol, 1 mL of the lecithin solution and 50 µL of the biotin-modified phosphatidylethanolamine solution were added, followed by sufficient mixing. The solvent was removed under reduced pressure by treating the thus-obtained mixed solution with a rotary evaporator in a 40°C bath for 1 hour or more under vacuum.

Next, a mixture of 50 µL of the 25 mg/mL GDH enzyme buffer solution, 50 µL of the 5 mg/mL DI enzyme buffer solution, and 50 µL of the 50 mM NADH buffer solution was added to the resultant product, and the resultant mixture was immersed in an ultrasonic bath for 30 to 60 seconds. During this treatment, a phospholipid film was mixed by scraping with a polytetrafluoroethylene scraper.

Next, the liposome solution prepared as described above is transferred to a 1.5 mL tube, and the remaining liposome solution was recovered with 50 µL of a 100 mM phosphate buffer solution, followed by two times of centrifugal washing with 1 mL of a 100 mM phosphate buffer solution under the conditions of 1500 g and 4 min.

The resultant GDH/DI/NADH-enclosed biotin-modified liposome was suspended in 600 µL of a 0.1M phosphate buffer solution, and amphotericin B was added so that the final concentration was 1 mM and mixed with vortex.

Consequently, the biotin-modified liposome in which GDH, DI, and NADH were enclosed and to which amphotericin B was bonded was formed.

The avidin-modified electrode was formed as follows:

To 200 mg of carbon paste (manufactured by B. A. S Co., Ltd.) produced by mixing graphite powder having a uniform particle diameter and paraffin oil used as an adhesive, 100 µL of a avidin agarose beads suspension and kneaded by light force on an agate mortar. Next, the thus-obtained avidin-containing carbon paste was dried in a desiccator for 1 hour or more and then kneaded into a pocket of the electrode having the pocket (recess) in the upper surface thereof. Then, the upper surface side of the electrode was pressed on copying paper in a circular motion to flatten the surface.

The avidin-modified electrode was formed as described above.

Next, the biotin-modified liposome in which GDH, DI, and NADH were enclosed and to which amphotericin B was bonded was immobilized on the avidin-modified electrode as follows:

First, the avidin-modified electrode formed as described above was immersed in 300 µL of the biotin-modified liposome formed as described above. Then, after incubation at room temperature for 1 hour, the electrode was washed two times with 500 µL of a 0.1 M phosphate buffer solution.

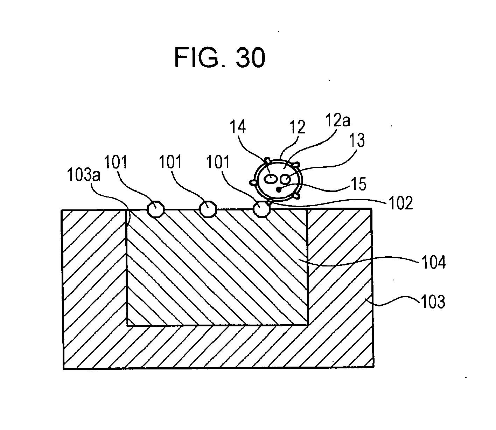

Fig. 30 shows an avidin-modified electrode on which biotin-modified liposome is immobilized, GDH, DI, and NADH being enclosed in the liposome and amphotericin B being bonded to the liposome. As shown in Fig. 30, a carbon paste electrode 104 is buried in a pocket 103a provided in the upper surface of an electrode 103, and avidin 101 is immobilized on the carbon paste electrode 104. In addition, the liposome 12 is bonded to the carbon paste electrode 104 by bonding the avidin 101 to the biotin 102 of the liposome 12. The enzymes 13 and 14 and the coenzyme 15 enclosed in the aqueous phase 12a within the liposome 12 are GDH, DI, and NADH, respectively.

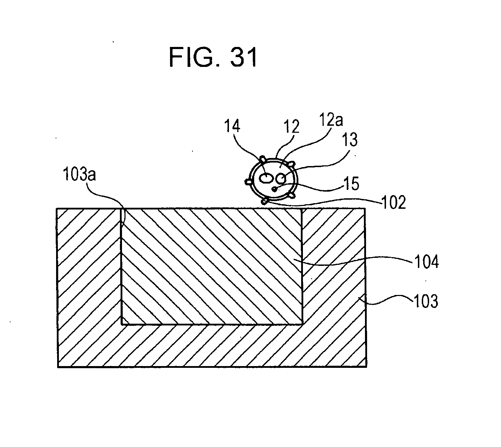

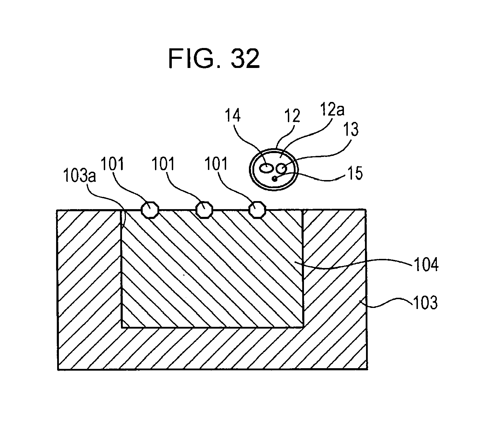

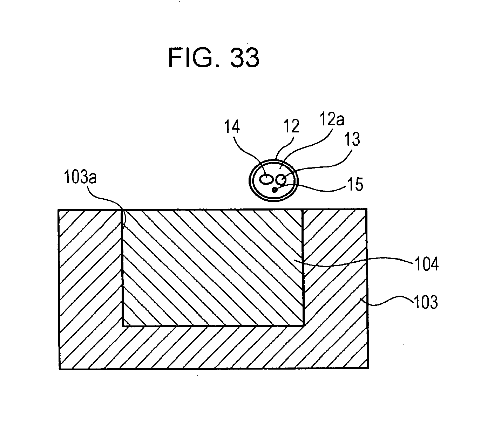

The electrochemical response of the liposome-immobilized electrode (hereafter abbreviated as "AviCPE-BioLipo" according to demand) formed as described above was measured. As a first comparative example, as shown in Fig. 31, the same liposome-immobilized electrode (hereinafter abbreviated as "CPE-BioLipo" according to demand) was formed by the same method as the above except that the avidin 101 was not immobilized on the carbon paste electrode 104. As a second comparative example, as shown in Fig. 32, the same liposome-immobilized electrode (hereinafter abbreviated as "AviCPE-Lipo" according to demand) was formed by the same method as the above except that the biotin 102 was not bonded to the liposome 12. As a third comparative example, as shown in Fig. 33, the same liposome-immobilized electrode (hereinafter abbreviated as "CPE-Lipo" according to demand) was formed by the same method as the above except that the avidin 101 was not immobilized on the carbon paste electrode 104, and the biotin 102 was not bonded to the liposome 12.

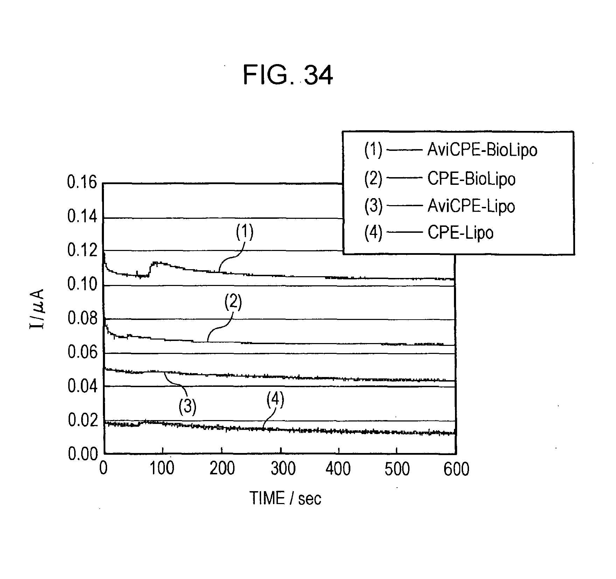

In a 2 mL tube, 180 µL of a 0.1 M phosphate buffer solution and 20 µL of a 10 mM Q0 buffer solution were added so that the final concentration of the Q0 buffer solution was 1 mM, and a platinum wire as a counter electrode, an AglAgCl reference electrode, and each of the above four types of liposome-immobilized electrodes (AviCPE-BioLipo, CPE-BioLipo, AviCPE-Lipo, and CPE-Lipo) were immersed in the tube, and chronoamperometry was performed at a potential set to +0.3 V with respect to the AglAgCl. After the start of measurement, 20 µL of a 1 M glucose solution was added so that the final concentration was 100 mM. The results of chronoamperometry are shown in Fig. 34. In Fig. 34, an offset of 0.03 µA is added to each of the current (I)-time curves shown, but the base current values are substantially the same. It is found from Fig. 34 that only when the liposome-immobilized electrode (AviCPE-BioLipo) in which the avidin 101 was immobilized on the carbon paste electrode 104 and the biotin 102 was bonded to the liposome 12 was used, relatively high current value response of 0.015 µA → 0.023 µA to addition of glucose is observed as compared with the other liposome-immobilized electrodes (CPE-BioLipo, AviCPE-Lipo, and CPE-Lipo).

As described above, according to the eighth embodiment, the enzymes 13 and 14 and the coenzyme 15 necessary for an enzyme reaction for decomposing glucose are enclosed in the liposome 12 having the pore 17 formed in the bimolecular lipid membrane by bonding the antibiotic 16. In addition, the avidin 101 is immobilized on the electrode 11, the biotin 102 is bonded to the liposome 12, and the liposome 12 is immobilized on the electrode 11 by bonding the avidin 101 immobilized on the electrode 11 and the biotin 102 bonded to the liposome 12. Therefore, in addition to the same advantages as in the first embodiment, the advantage of permitting the liposome 12 to be easily, securely, strongly immobilized on the electrode 11 can be obtained. The liposome-immobilized electrode is suitable for use as, for example, a negative electrode of a biofuel cell.

### (9. Ninth embodiment)

### [Enzyme-immobilized electrode]

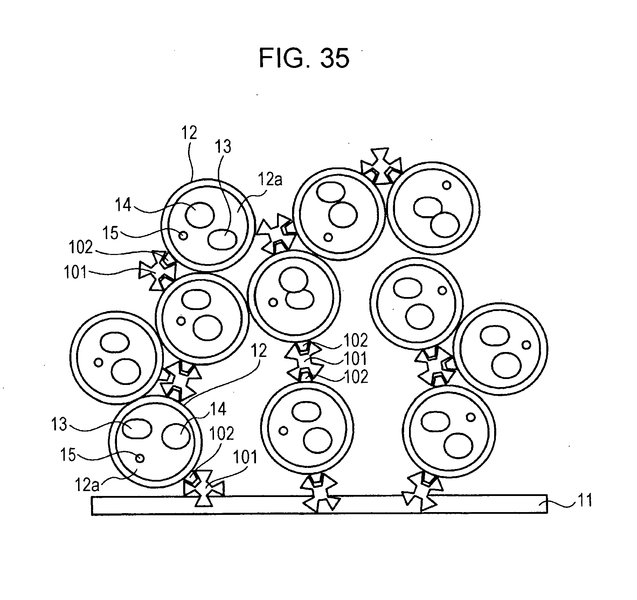

Fig. 35 shows an enzyme-immobilized electrode according to the ninth embodiment. In the enzyme-immobilized electrode, glucose is used as a substrate.

As shown in Fig. 35, in the enzyme-immobilized electrode, multiple layers of liposome 12 are immobilized on a surface of an electrode 11 using the matter that avidin 101 has four biotin-binding pockets. That is, like in the eighth embodiment, the liposome 12 is immobilized on the electrode 11 by irreversible specific bonding between the avidin 101 immobilized on the surface of the electrode 11 and the biotin 102 bonded to the liposome 12. In addition, in this case, a plurality of molecules of the biotin 102 (in the example shown in Fig. 35, two) are bonded to the liposome 12 immobilized on the surface of the electrode 11. The biotin 102 not used for immobilizing the liposome 12 immobilized on the surface of the electrode 11 is bonded to the avidin 101 not immobilized on the surface of the electrode 11. The avidin 101 not immobilized is bonded to one or two or more biotins 102 bonded to other liposomes 12, thereby bonding the liposomes 12 each other. Consequently, multiple layers of the liposomes 12 are immobilized on the surface of the electrode 11. The other conditions are the same as in the eighth embodiment.

### [Method for producing enzyme-immobilized electrode]

In order to produce the enzyme-immobilized electrode, the liposome 12 is immobilized on the electrode 11 by the same method as for the enzyme-immobilized electrode according to the eighth embodiment, and then liposome 12 is laminated by, for example, adding avidin 101 or avidin agarose. In this case, the thickness of laminated layers of liposome 12 and the density of liposome 12 can be controlled by adjusting the amount of the avidin added and the ratio of a biotin-modified lipid in the bimolecular lipid membrane constituting liposome 12.

According to the ninth embodiment, the same advantages as in the eighth embodiment can be obtained.

Although the embodiments and the examples of the present invention are specifically described above, the present invention is not limited to the above-described embodiments and examples, and various modifications can be made on the basis of the technical idea of the invention.

For example, the numerical values, structures, configurations, shapes, materials, and the like described in the above-described embodiments and examples are only examples, and numerical values, structures, configurations, shapes, materials, and the like different from these can be used according to demand.

In addition, the method for immobilizing liposome on an electrode using specific bonding between avidin and biotin according to the present invention is a method effective for a case where the bimolecular lipid membrane constituting liposome does not have pores permeable to glucose. For example, when the electrode as a negative electrode of a fuel cell, a fuel is not limited to glucose, and various fuels such as alcohols can be used.

### Reference Signs List

- 11: electrode
- 12: liposome
- 13, 14: enzyme
- 15: coenzyme
- 16: antibiotic
- 17: pore
- 21: negative electrode
- 22: positive electrode
- 23: electrolyte layer
- 41, 42: Ti current collector
- 43, 44: fixing plate
- 47: load
- 81, 90: cell membrane
- 85: outer membrane
- 86: inner membrane
- 101: avidin
- 102: biotin

## Claims

1. A fuel cell configured to have a structure in which a positive electrode and a negative electrode face each other with a proton conductor provided therebetween, and to extract electrons from a fuel using an enzyme,
wherein at least one type of enzyme is enclosed in liposome; and
a bimolecular lipid membrane constituting the liposome has one or more pores permeable to glucose.

2. The fuel cell according to Claim 1, configured to extract electrons from the fuel using the enzyme and a coenzyme, wherein at least one type of enzyme and at least one type of coenzyme are enclosed in the liposome.

3. The fuel cell according to Claim 2, wherein the pores are formed by an antibiotic bonded to the bimolecular lipid membrane.

4. The fuel cell according to Claim 3, wherein the antibiotic is amphotericin B.

5. The fuel cell according to Claim 3, wherein the antibiotic is ionophore.

6. The fuel cell according to Claim 1, wherein the liposome is immobilized on the negative electrode.

7. The fuel cell according to Claim 6, wherein a first material immobilized on the negative electrode and a second material bonded to the liposome are bonded to each other.

8. The fuel cell according to Claim 7, wherein a combination of the first material and the second material is avidin and biotin, an antigen and an antibody, protein A and immunoglobulin IgG, protein G and immunoglobulin IgG, sugar molecule and lectin, DNA and complementary strand DNA, glutathione and glutathione S-transferase, heparin and heparin-binding molecule, hormone and hormone receptor, or a carboxylic acid and an imide.

9. The fuel cell according to Claim 7, wherein at least two liposomes are bonded to each other.

10. A method for producing a fuel cell having a structure in which a positive electrode and a negative electrode face each other with a proton conductor provided therebetween so that electrons are extracted from a fuel using an enzyme, the method comprising a step of forming one or more pores permeable to glucose in a bimolecular lipid membrane constituting liposome after at least one type of enzyme is enclosed in the liposome.

11. The method for producing a fuel cell according to Claim 10, wherein the fuel cell extracts electrons from a fuel using the enzyme and a coenzyme, and at least one type of enzyme and at least one type of coenzyme are enclosed in the liposome.

12. An electronic apparatus comprising one or more fuel cells, wherein at least one of the fuel cells is configured to have a structure in which a positive electrode and a negative electrode face each other with a proton conductor provided therebetween, and to extract electrons from a fuel using an enzyme;
at least one type of enzyme is enclosed in liposome; and
a bimolecular lipid membrane constituting the liposome has one or more pores permeable to glucose.

13. The electronic apparatus according to Claim 12, wherein the fuel cell is configured to extract electrons from the fuel using the enzyme and a coenzyme, and at least one type of enzyme and at least one type of coenzyme are enclosed in the liposome.

14. An enzyme-immobilized electrode,
wherein liposome in which at least one type of enzyme is enclosed is immobilized; and
a bimolecular lipid membrane constituting the liposome has one or more pores permeable to glucose.

15. The enzyme-immobilized electrode according to claim 14, wherein at least one type of enzyme and at least one type of coenzyme are enclosed in the liposome.

16. A biosensor using an enzyme,
wherein at least one type of enzyme is enclosed in liposome; and
a bimolecular lipid membrane constituting the liposome has one or more pores permeable to glucose.

17. An energy-conversion element using liposome in which at least one type of enzyme is enclosed, wherein a bimolecular lipid membrane constituting the liposome has one or more pores permeable to glucose.

18. A cell, wherein a bimolecular lipid membrane constituting a cell membrane has one or more pores permeable to glucose.

19. An organelle, wherein a bimolecular lipid membrane constituting a cell membrane has one or more pores permeable to glucose.

20. A bacterium, wherein a bimolecular lipid membrane constituting a cell membrane has one or more pores permeable to glucose.
